(19) Europäisches Patentamt
European Patent Office
Office européen des brevets

(11) Veröffentlichungsnummer: **0 070 436**
**B1**

(12) # EUROPÄISCHE PATENTSCHRIFT

(45) Veröffentlichungstag der Patentschrift: **26.02.86**

(51) Int. Cl.[4]: **C 08 J 9/00**, C 07 C 87/54, C 07 D 279/20, C 07 D 279/30, C 08 L 75/04

(21) Anmeldenummer: **82105902.9**

(22) Anmeldetag: **02.07.82**

(54) **Stabilisatorhaltige Reaktivkomponenten für PU-Schaumstoffe, neue Stabilisatoren und ein Verfahren zur Herstellung der Stabilisatoren.**

(30) Priorität: **14.07.81 DE 3127750**

(43) Veröffentlichungstag der Anmeldung: **26.01.83 Patentblatt 83/4**

(45) Bekanntmachung des Hinweises auf die Patenterteilung: **26.02.86 Patentblatt 86/9**

(84) Benannte Vertragsstaaten: **BE DE FR GB IT NL SE**

(56) Entgegenhaltungen:
**US - A - 3 798 184**
**US - A - 4 007 230**
**US - A - 4 146 687**
**US - A - 4 235 975**
**US - A - 4 275 173**

**CHEMICAL ABSTRACTS, Band 77, Nr. 23, 4. Dezember 1972, Seite 71, Nr. 153541s, Columbus Ohio (USA)**
**G. Geuskens, "DEGRADATION AND STABILISATION OF POLYMERS" (1975), S. 71-73**
**R. Gächter/H. Müller, "TASCHENBUCH DER KUNSTSTOFF-ADDITIVE" (1979), S. 7-9**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Buysch, Hans-Josef, Dr., Brandenburger Strasse 28, D-4150 Krefeld (DE)**
Erfinder: **Ilger, Hans-Walter, Dr., Im Tentefeld 27, D-5064 Roesrath (DE)**
Erfinder: **Dörner, Karl Heinz, Weilersgrund 26, D-5024 Pulheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

EP 0 070 436 B1

**Beschreibung**

Die Erfindung betrifft stabilisatorhaltige Reaktivkomponenten zur Herstellung von nicht oder wenig kernverfärbenden Polyurethanschaumstoffen, welche durch einen stabilisierenden Zusatz von monomeren und/oder oligomeren Derivaten der Diphenylamin-Reihe, einschließlich der Phenothiazin-Reihe gekennzeichnet sind; ferner neue Stabilisatoren der Diphenylamin- und/oder Phenothiazin-Reihe, sowie ein Verfahren zu ihrer Herstellung.

Bekanntlich werden Polyurethanschaumstoffe mit den verschiedensten physikalischen Eigenschaften nach dem Isocyanat-Polyadditions-Verfahren aus Verbindungen mit mehreren aktiven H-Atomen, insbesondere OH- und COOH-Gruppen enthaltenden Verbindungen und Polyisocyanaten, gegebenenfalls unter Mitverwendung von Wasser und/oder anderen Treibmitteln, Aktivatoren, Emulgatoren, Schaumstabilisatoren und Antioxidantien seit langem in großem Maße technisch hergestellt (Vieweg/Höchtlen, Kunststoffhandbuch, Bd. VII, Polyurethane, Carl-Hanser-Verlag, München 1966). Auf diese Weise lassen sich je nach Art der eingesetzten Komponenten sowohl elastische als auch starre Schaumstoffe bzw. alle dazwischen liegenden Varianten herstellen.

Polyurethanschaumstoffe werden vorzugsweise durch Vermischen flüssiger Komponenten hergestellt, wobei man die miteinander umzusetzenden Ausgangsmaterialien entweder gleichzeitig vermischt oder aber zunächst aus Polyhydroxylverbindungen, wie Polyalkylenglykolether oder OH-Gruppen aufweisenden Polyestern mit einem Über-schuß an Polyisocyanat ein NCO-Gruppen enthaltendes Voraddukt herstellt, das dann in einem zweiten Schritt, z.B. mit Wasser, in den Schaumstoff übergeführt wird.

Bei der Verschäumung, insbesondere dann, wenn es sich um Schaumstoffe mit niedrigen Raumgewichten handelt oder wenn eine verhältnismäßig große Isocyanatmenge oder Wassermenge verwendet wird, treten unerwünschte bräunliche bis tiefbraune Verfärbungen im Schaumblock auf, vornehmlich im Kern des Blockes, wo die Temperatur bei der Verschäumung am höchsten steigt und am längsten erhalten bleibt. Derartige Kernverfärbungen sind in der Regel mit einer Schädigung des Schaumstoffes verbunden, die sich in einer erheblichen Minderung des mechanischen Eigenschaftsniveaus ausdrückt. Führt schon eine solche Verfärbung und damit einhergehende Schädigung zu unbrauchbaren Schaumstoffen und wirtschaftlichen Nachteilen, so ist doch die eigentliche und am meisten gefürchtete Gefahr bei der Verschäumung im groß-industriellen Maßstab in der Selbstentzündung großer Schaumstoffmassen zu sehen, die bei zur Kernverfärbung neigenden Produkten in unkontrollierbarer Weise eintreten und zu Großbränden mit schwerwiegenden Folgen führen kann. Diese unerwünschten Vorgänge können durch bei der Verschäumung mitverwendete Zusatzstoffe auch verstärkt werden. Zu diesen Stoffen gehören z.B. tertiäre Amine, die als Katalysatoren, Halogenkohlenwasserstoffe, die als Treibmittel und halogenhaltige Phosphorsäureester, die als Flammschutzmittel eingesetzt werden.

Man hat daher schon eine Reihe von Vorschlägen gemacht, wie Kernverfärbung und Kernverbrennung unterdrückt werden können.

Zunächst hat man durch Zusatz von sterisch gehinderten Phenolen (vgl. US-PS 3 494 880 bzw. 3 437 694) gewisse, aber durchaus unzureichende Fortschritte erzielt. Ähnliches gilt für die Verwendung von Phenothiazin (vgl. US-PS 3 214 397 und 4 143 219). Eine Verbesserung wurde auch erzielt durch Einsatz einer Kombination von Dioctyl-diphenylamin und 2,6-Di-tert.-butyl-p-kresol (Ionol) (vgl. US-PS 3 567 664 und 3 637 865). Schließ-lich kann man einen Teil des Ionols durch Phenothiazin ersetzen, wobei die stabilisierende Wirkung in der Regel nicht verändert, in einigen Fällen geringfügig angehoben wird (US-PS 40 10 211).

In der US-PS 4 146 687 werden flammgeschützte Polyurethanschaumstoffe beschrieben, welche halogenhaltige Phosphorsäurealkylester als Flammschutzmittel und - zur Verhinderung von durch die Flammschutzmittel hervorgerufene Verfärbungen zusätzlich eine Mischung aus Phenothiazin und bestimmten aromatischen Aminantioxidantien, z. B. dem 4,4'-Dioctyl-diphenylamim, dem N-Isopropyl-N'-phenyl-paraphenylendismin oder dem Dirhenylamin/Aceton Kondensationsprodukt, enthalten. Phenothiazin zeigt in PU-Schaumstoffen jedoch eine starke Rosarotverfärbung, aber nur eine geringe Anti-Kernverfärbungswirkunq. Das Diphenylamin/Aceton Kondensationsprodukt verfärbt den gesamten Schaumstoff sehr stark. Auch Kombinationen aus den Aminen und Phenothiezin ergeben sehr starke Kernverfärbungen.

Aufgabe der Erfindung war daher, stabilisierte Resktiv-Komponenten zur Herstellung von Polyurethanschaumstoffen mit einer erheblich verbesserten Stabilisierung gegen Kernverfärbung, besonders Kernverbrennung und Selbstentzündung bei der Schaumstoffbildung zu entwikkeln.

Es wurde nun gefunden, daß man derartig stabilisierte Polyurethan-Schaumstoff-Reaktivsysteme und auch stabilisierte Polyurethan-Schaumstoffe erhält, wenn man während der Schaumstoffbildung als Stabilisatoren Verbindungen der Diphenylamin-Reihe inclusive der Phenothiazin-Reihe als Stabilisatorbestandteile in den Reaktivkomponenten verwendet.

Gegenstand der Erfindung sind somit stabilisatorhaltige Reaktivkomponenten zur Herstellungvon nicht oder weniü kernverfärbenden Polyurethan-Schaumstoffen auf der Basis von Polyisocyanaten, Polyolen sowie gegebenenfalls Wasser, Treibmitteln, Katalysatoren, gegebenenfalls weiteren Stabilisatoren und üblichen Zuschlagstoffen, dadurch gekennzeichnet, daß sie als Stabilisatoren monomere und/oder oligomere Derivate der Diphenylamin-Reihe einschließlich der Phenothiazin-Reihe in stabilisierenden Mengen von 0,02 bis 5 Gew.-% an Verbindungen der allgemeinen Struktur 1

I . 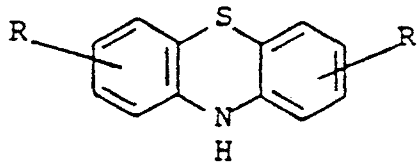 ,

in der
R = $C_7$-$C_{18}$-Aralkyl, vorzugsweise $C_7$-$C_{12}$-Aralkyl bedeutet (die Zahl der C-Atome gibt die Gesamtzahl im Arylrest inclusive seiner Alkylsubstitution an);
der allgemeinen Struktur II

II 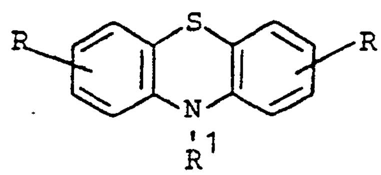 ,

in der
$R^1$ = $C_1$-$C_{18}$-Alkyl, vorzugsweise $C_1$-$C_6$-Alkyl, $C_5$-$C_{12}$-Cycloalkyl und -Cycloalkenyl (vorzugsweise $C_5$/$C_6$-Cycloalkyl), $C_7$-$C_{18}$-Aralkyl (vorzugsweise $C_7$-$C_{12}$-Aralkyl), die gegebenenfalls durch OH-, SH-, Ether-, Thioether-, Carbonester-, Carbonamid- und Carboxyl-Gruppen substituiert oder durch solche Gruppen (außer OH, SH, COOH) und olefinische Doppelbindungen unterbrochen sein können,
ferner ein Rest

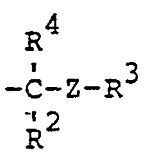

ist,
worin $R^2$, $R^3$ und $R^4$ gleich oder verschieden sind und
H, $C_1$-$C_{12}$-Alkyl, $C_5$-$C_{12}$-Cycloalkyl und -Cycloalkenyl (vorzugsweise $C_5$- und $C_6$-Cycloalkyl), $C_7$-$C_{12}$-Aralkyl, (vorzugsweise $C_7$-$C_{10}$-Aralkyl), $R_2$ außerdem gegebenenfalls substituiartes Aryl ist und mit $R^4$ und dem zentralen C-Atom zusammen einen 5 - 12 (vorzugsweise 5 oder 6-) gliedrigen aliphatischen Ring bildet,
Z O, S, NH, $NR^5$, worin $R^5$ = $R^2$ oder ein Rest der Formel CO-A-$R^2$ ist in der A eine einfache Bindung, S, O, NH oder $NR^2$ bedeutet, wobei hier und folgend für $R^2$ die Ringbildung mit $R^4$ entfällt,
Z mit $R^3$ zusammen den Rest

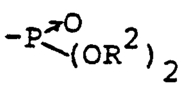 und

$R^1$ ferner den Rest

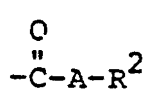

darstellt,
der allgemeinen Struktur III

III 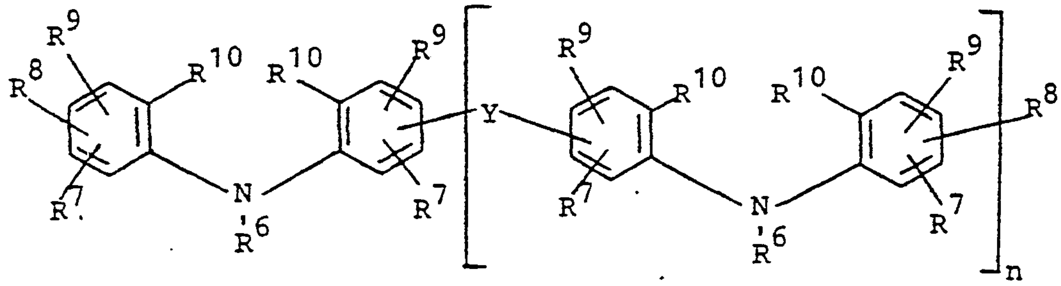

in der
$R^6$ Wasserstoff oder $R^1$,
$R^7$, $R^9$ und $R^{10}$ gleich und verschieden sind und H, $CH_3$ oder $C_2H_5$ bedeuten (vorzugsweise H),
$R^8$ = H, Benzyl-, Styryl-, α-Methylstyryl-, tert.-Butyl-, tert.-Amyl-,

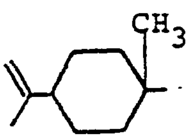 , 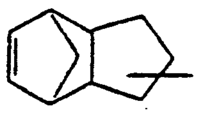 , 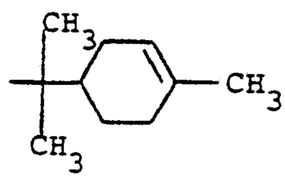 ,

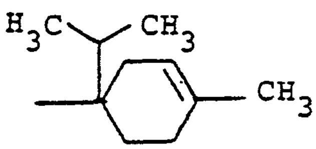 , 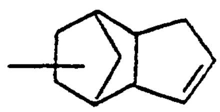 , 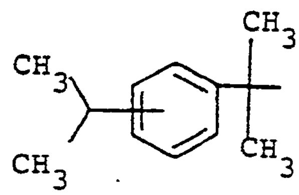

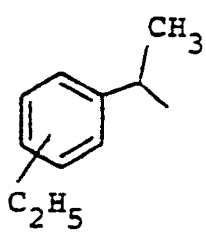 , 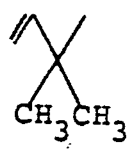 ,

und weniger bevorzugt

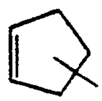, 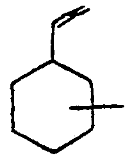, 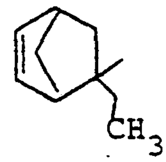,

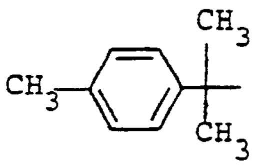, 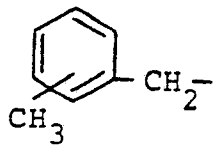, 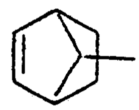,

Isononyl-, Cyclohexyl- und Methylcyclohexyl- bedeuten,

$$Y = -\overset{\overset{R^7}{|}}{\underset{|}{C}}-R^{11},$$

wobei $R^7$ die obengenannte Bedeutung hat, vorzugsweise aber H ist und wobei $R^{11}$ $C_1$-$C_7$- (vorzugsweise $C_1$-$C_4$-) Alkyl, Cyclohexyl, Cyclohexenyl und Aryl ist, weiter

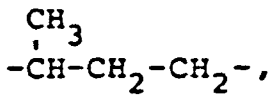, 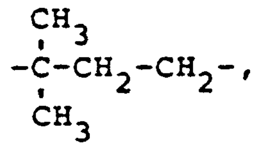, 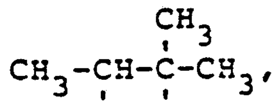,

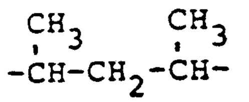, 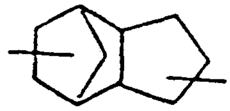, 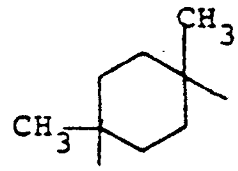, 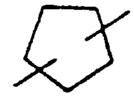,

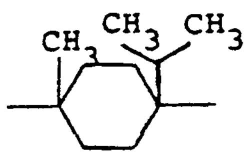, 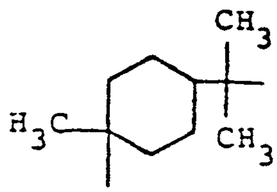, 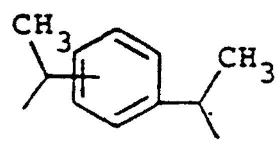,

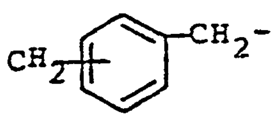, 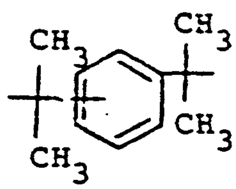, 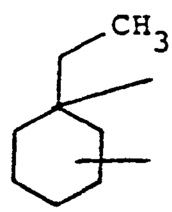, 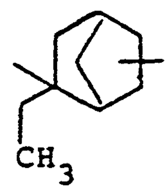.

und bis zu 60 Mol-% auch -S-, $-CH_2-$, $-CH_2-S-CH_2-$oder $-CH_20-CH_2-$ (vorzugsweise $-CH_2-$) bedeutet und n eine ganze Zahl von 1 bis 29, vorzugsweise von bis 19 darstellt;
jedoch ausgeschlossen Produkte entsprechend der Umsetzung von Diphenylamin und Aceton ($R^6$ bis $R^{10}$ = H;

Y = 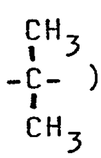 );

der allgemeinen Struktur IV:

IV 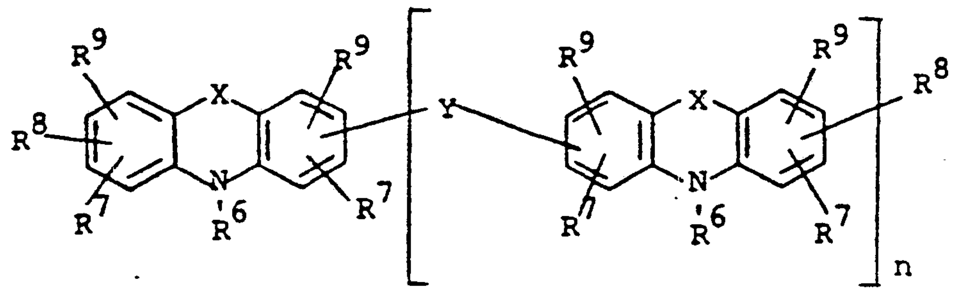

in der
X -S- bedeutet;
und der allgemeinen Struktur V

V

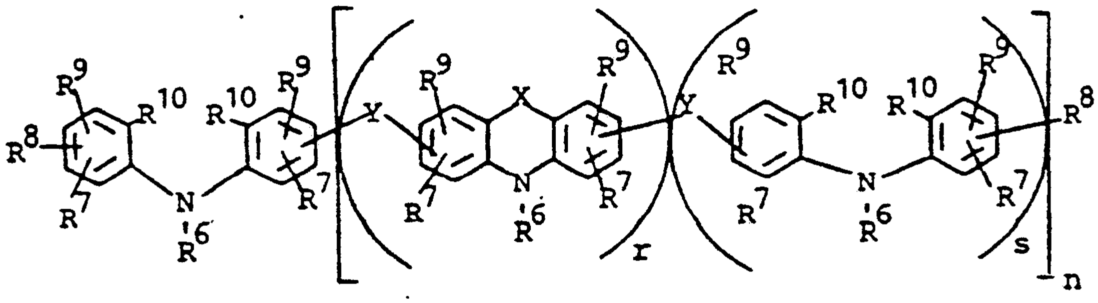

in der
r und s ganze Zahlen von 1 bis 29, vorzugsweise von 1 bis 9, sind,
enthalten.

Mit diesen Verbindungen geschützte Polyurethan-Reaktivkompositionen können mit entsprechend hohen Isocvanatbzw. Wasseranteilen auch zu Schaumstoffen mit sehr niedrigen Dichten verarbeitet werden, wobei mit großer Sicherheit Kernverbrennungen und Selbstentzündungen ausgeschlossen werden. An vergleichbaren Ansätzen mit herkömmlicher Stabilisierung werden zumindest tiefbraune Kernverfärbungen, häufig Kernverbrennungen oder gar Abbrennen des Blockes beobachtet.

Die erfindungsgemäß den Ausgangsstoffen bzw. Stoffmischungen zuzusetzenden Substanzen werden in stabilisierenden Mengen, z.B. von 0,02 bis 5,0 Gew.-%, bevorzugt 0,05 bis 2,5 Gew.-%, besonders aber 0,07 bis 1,5 Gew.-%, bezogen auf die gesamte Polyurethanmasse, eingesetzt. Sie können als Konzentrat in einem inerten Lösungsmittel, gelöst in den für die Polyurethanherstellung benötigten Zuschlägen wie Katalysatoren oder Flammschutzmitteln, oder in den eigentlichen Reaktionspartnern, den Polyoloder Polyisocyanat-Komponenten zugegeben werden.

Die monomeren und besonders die oligomeren Gemische der Stabilisatoren der allgemeinen Formeln III bis V werden bevorzugt; ganz besonders bevorzugt sind jedoch Verbindungen der allgemeinen Formeln IV und V. Die besten Ergebnisse werden mit Stabilisatoren des Typus IV erhalten.

Diese Stabilisatoren können auch in Kombination mit anderen bekannten Stabilisatoren aus der Reihe der sterisch gehinderten Phenole, der Phosphorigsäureester, der Phosphine und der Thioether eingesetzt werden, wobei auch synergistische Steigerungen der Wirksamkeit festgestellt werden können.

Geeignete Substanzen für Stabilisator-Kombinationsmischungen sind beispielsweise:
Phenole der Formel

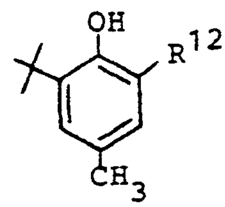

$R^{12}$ = Methyl, tert.-Butyl, tert.-Amyl, Cyclohexyl, Cyclopentyl;
Phosphine und Phosphite der Formeln
$P(SR^{13})_3$, $P(OR^{13})_3$,
und Thioether der Formeln
$S(R^{13})_2$ und $S(CH_2CH_2COOR^{13})_2$,
in denen
$R^{13}$ einen $C_2$-$C_{20}$-Alkyl-, $C_5$-$C_{12}$-Cycloalkyl- oder $C_6$-$C_{10}$-Arylrest darstellen,
namentlich z.B.
2,6-Di-tert.-butyl-p-kresol,
2-tert.-Butyl-6-cyclohexyl-p-kresol,
Triphenylphosphin,
Tris-(p-N,N-dimethylphenyl)-phosphin,
Di-(nonyl-phenyl)-phosphit,
Tri-(nonyl-phenyl)-phosphit,
Tri-(2,4-di-tert.-butyl-phenyl)-phosphit,
Triphenylphosphit,
Tributylphosphit,
Tris-dipropylenglykol-phosphit und
Di-lauryl-thiodipropionat.

Gegenstand der Erfindung sind auch neue Verbindungen der allgemeinen Struktur III

III 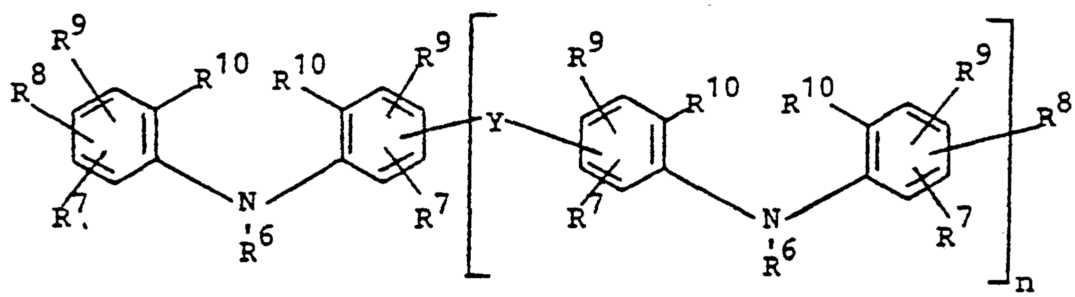

in der
$R^6$ Wasserstoff oder $R^1$,
$R^7$, $R^9$ und $R^{10}$ gleich oder verschieden sind und H, $CH_3$ oder $C_2H_5$ bedeuten (vorzugsweise H),
$R^8$ = H, Benzyl-, Styryl-, α-Methylstyryl-, tert.-Butyl-, tert.-Amyl-,

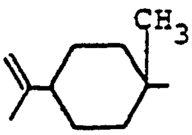 , 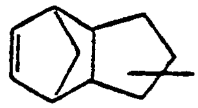 , 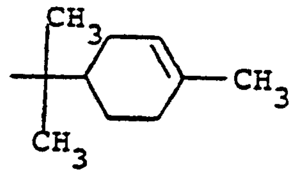 ,

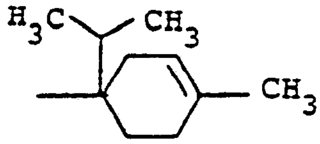 , 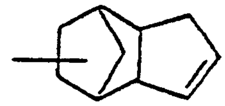 , 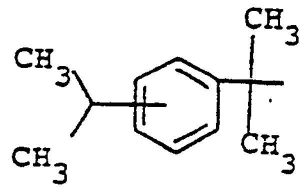 ,

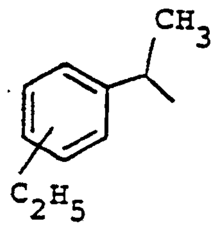 , 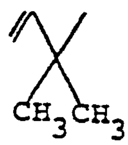 ,

und weniger bevorzugt

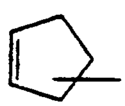 , 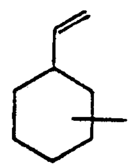 , 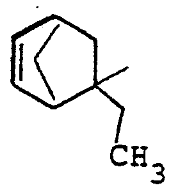 ,

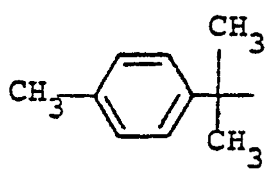 , 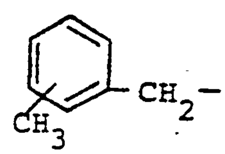 , 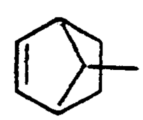 ,

Isononyl-, Cyclohexyl- und Methylcyclohexyl- bedeuten,

Y = 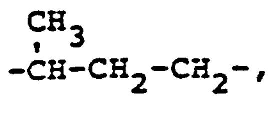, 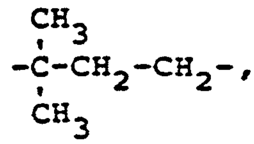, 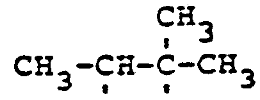,

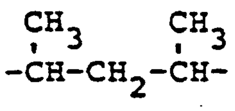, 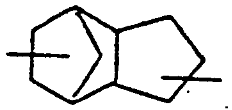, 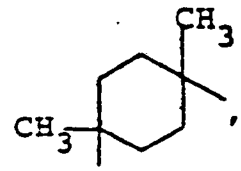, 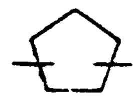,

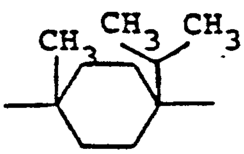, 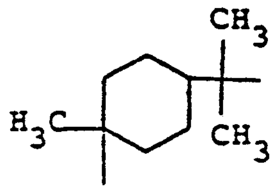, 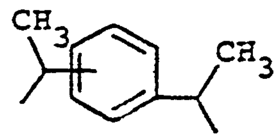, 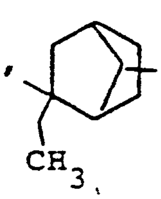,

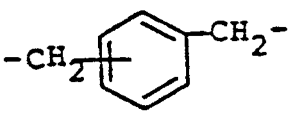, 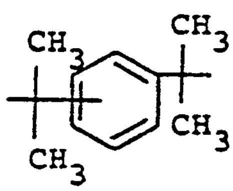, 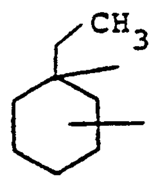

und bis zu 60 Mol-% auch -S-, $-CH_2-$, $-CH_2-S-CH_2-$oder $-CH_20-CH_2-$ (vorzugsweise $-CH_2-$) und n eine ganze Zahl von 1 bis 29 (vorzugsweise von 1 bis 19) darstellt, jedoch ausgeschlossen Verbindungen, bei denen $R^6$ bis $R^{10}$ = H und

Y = 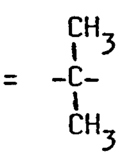 ist;

der allgemeinen Struktur IV

IV 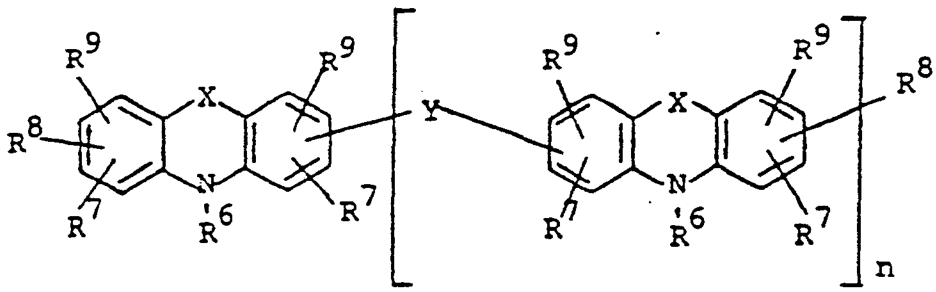

in der

Y = 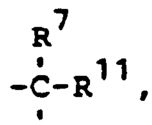,

wobei $R^7$ die obengenannte Bedeutung hat vorzugsweise aber H ist und wobei $R^{11}$ $C_1$-$C_7$- (vorzugsweise $C_1$-$C_4$-) Alkyl, Cyclohexyl, Cyclohexenyl und Aryl ist, weiter die unter III. aufgeführte Bedeutung hat,
X - S - bedeutet
und der allgemeinen Struktur V

V 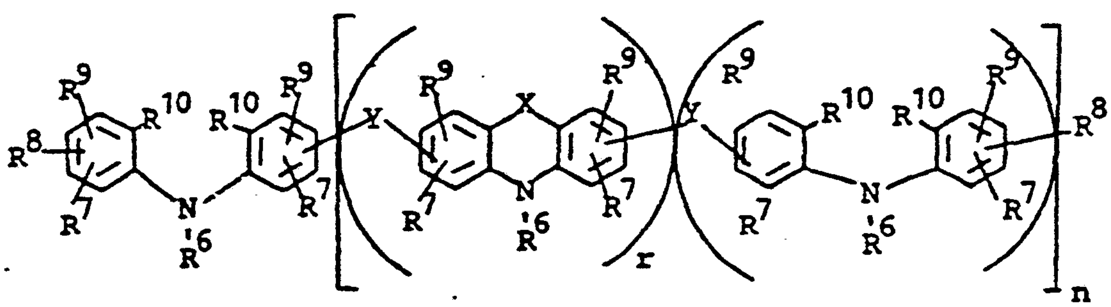

in der
r und s ganze Zahlen von 1 bis 29 (vorzugsweise von 1 bis 19) darstellen.

Verbindungen der Struktur 1 sind durch Alkylierung von Phenothiazin nach an sich bekannten Verfahren zugänglich, z.B. Alkylierung mit α-Methylstyrol oder Styrol entsprechend der Japanischen Patentanmeldung 72 19 088.

Verbindungen der Struktur II lassen sich durch Substitution des H-Atoms am Stickstoff aus Verbindungen der Struktur 1 herstellen. Die Substitution kann durch Alkylierung mit Alkylhalogeniden, Benzylhalogeniden und analogen Verbindungen durch Oxalkylie;ung mit Ethylenoxid oder Propylenoxid oder auch durch Acylierung mit Carbonsäurehalogeniden, Chlorkohlensäureestern und anderen Acylierungsmitteln erfolgen.

Ein weiterer Gegenstand der Erfindung ist ein Verfahren zur Herstellung von monomeren Verbindungen oder oligomeren Gemischen aus Verbindungen der Strukturen III bis V, das dadurch gekennzeichnet ist, daß man aromatische Amine der Formel

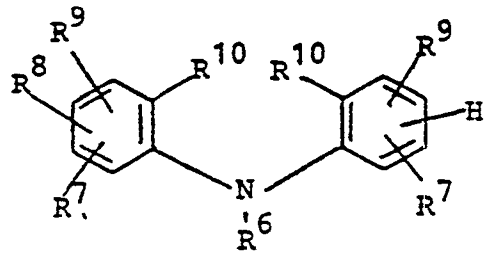

und

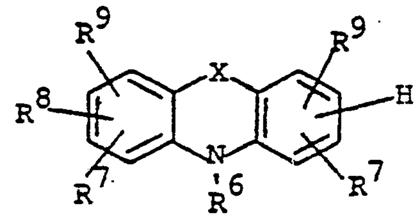

(Bedeutung der Reste wie vorstehend)
mit bifunktionellen Verbindungen der Formeln
Hal-Y-Hal (Hal = Halogen)
HO-Y-OH
$R^7O$-Y-$OR^7$
$R^7COO$-Y-$OCOR^7$
bzw. aus diesen durch Abspaltung des Restes HOH, $HOR^7$, $HOCOR^7$ oder H-Hal sich bildenden Bisolefinen in Gegenwart von starken Säuren mit einem $pk_s$-Wert kleiner als 2 bei Temperaturen zwischen 50 - 300°C, vorzugsweise 120 - 250°C umsetzt und die Reste $R^8$ und $R^6$ (je ungleich H) vor, während oder nach der obigen Umsetzung der Amine einführt.

Entsprechend stellt man die erfindungsgemäßen neuen Stabilisatoren der Struktur III bis V durch Umsetzung der aromatischen Amine Diphenylamin, Phenothiazin oder der entsprechend mit $R^6$, $R^7$ und $R^9$ substituierten Amine in reiner Form oder als Gemisch mit den der Verknüpfungseinheit Y (siehe allgemeine Formel) zugrundeliegenden bifunktionellen Verbindungen, d.h. den entsprechenden Hydroxyverbindungen, Ethern, Halogeniden, Olefinen und Estern um.

Am Beispiel der Verknüpfung durch die Brücke VI

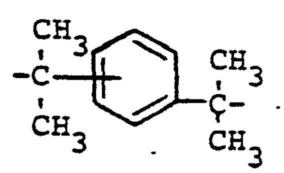

(VI)

(VI) sei dies erläutert:

Um die obige Y-Einheit VI zur Herstellung der Substanzen III - V einzuführen, können folgende zugrundeliegenden Verbindungen eingesetzt werden:

Olefin

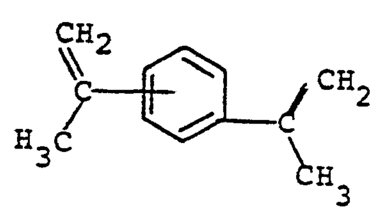

Hydroxyverbindung

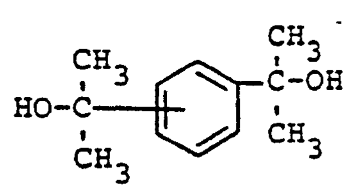

Ether

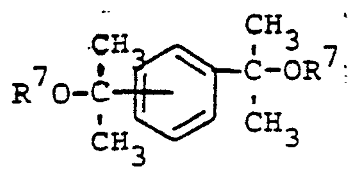

Halogenid

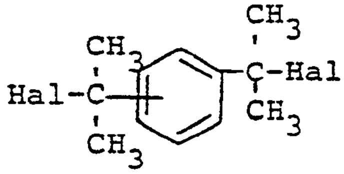

(Hal = Cl, Br)

Ester

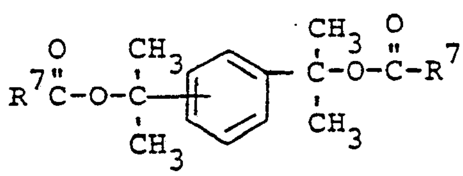

Diese Verbindungen wirken entweder direkt, wie das obige Olefin, alkylierend auf den aminischen Aromaten ein oder indirekt nach Abspaltung des Restes $H_20$, $R^7OH$, HCl ode; HBr oder $R^7COOH$ und bewirken die Verknüpfung zweier aromatischer Amine und zwar jeweils in ortho- oder para-Stellung zum Stickstoffatom.

Der Rest $R^8$ wird entweder während der oben beschriebenen Umsetzung (ebenfalls in ortho- oder para-Stellung während der Umsetzung) durch Anwendung eines Überschusses der Y zugrundeliegenden alkylierenden Verbindung eingeführt. Man kann ihn auch nachträglich einführen, indem man Substanzen der Formel III bis V, die an der Stelle $R^8$ noch Wasserstoff besitzen mit einer entsprechenden alkylierenden Verbindung wie Styrol, α-Methylstyrol, Benzylalkohol, Cyclohexen, Isononylen, Isobutylen unter den oben beschriebenen-Synthesebedingungen reagieren läßt. Oder man setzt von vornherein in einem Molverhältnis entsprechend dem angestrebten Wert von n eine Verbindung zu, die den Rest $R^8$ (nicht H) schon besitzt, also

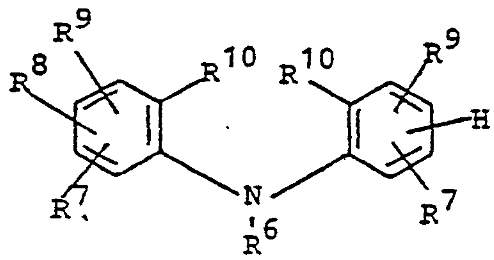

oder

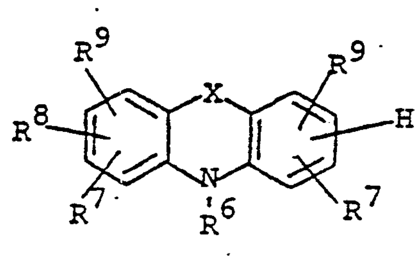

d.h. für den Fall, daß n im Durchschnitt 3 sein soll, wird pro Mol aromatisches Amin mit $R^8 = H$ 1 Mol aromatisches Amin mit einem Substituenten $R^8$ nicht gleich H eingesetzt.

Der Rest $R^6$ (ungleich H) kann nach den gleichen Verfahrensvarianten eingeführt werden wie $R^8$ (ungleich H). Für den Fall, daß $R^6$ nicht Alkyl oder Cycloalkyl ist, empfiehlt sich jedoch eine nachträgliche Einführung unter milderen und veränderten Bedingungen, da diese anderen Reste unter den Bedingungen der Aufbaureaktion möglicherweise wieder abgelöst werden. So kann beispielsweise eine Benzylgruppe wandern und wird daher zweck-mäßig später durch schonende Alkylierung, z.B. des Natriumsalzes der Verbindung III bis V (Substitution des NH-Wasserstoffs durch Na) mittels Benzylchlorid mit dem Stickstoffatom verknüpft.

Aus den gleichen Gründen wird $R^6$ auch dann nachträglich eingeführt, wenn $R^6$ den Rest

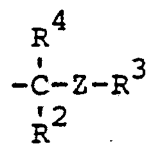

darstellt. Dieser Rest entsteht nach an sich bekannten Verfahren, beispielsweise durch Kondensation der Verbindungen der Struktur III bis V ($R^6 = H$) mit Aldehyden und Ketonen der Formel $R^2COR^4$ und Verbindungen der Formel $HZR^3$ unter milden Bedingungen, z.B. Raumtemperatur bis 100°C, in Gegenwart von Basen oder sehr geringer katalytischer Mengen auch schwacher Säuren.

Eine nachträgliche Substitution des NH-Wasserstoffatomes ist vorteilhaft, wenn der Rest $R^6$-CO-A-$R^2$ sein soll. Ein solcher Rest bewirkt nämlich eine gewisse Desaktivierung der aromatischen Amine gegenüber Alkylierungsreagentien, so daß bei der Aufbaureaktion unnötig scharfe Bedingungen erforderlich wären.

Die Umwandlung der Verbindungen III - V ($R^6 = H$) in solche mit $R^6$ = CO-A-$R^2$ geschieht nach den bekannten Acylierungsmethoden, beispielsweise mit Säurechloriden nach Einhorn oder Schotten-Baumann oder thermische Halogenwasserstoffabspaltung oder mit Säureanhydriden.

Die Reaktionspartner aromatisches Amin und Verknüpfungsreagenz werden in einem Molverhältnis von 5:1 bis 1:5, vorzugsweise 2:1 bis 1:2, bevorzugt 1,5:1 bis 1:1,5, eingesetzt. Überschüssiges Ausgangsmaterial kann bei der Aufarbeitung destillativ entfernt werden.

Die Umsetzung der aminischen, gegebenenfalls substituierten Grundkörper mit diesen der Brücke Y zugrundeliegenden Substanzen erfolgt bei Temperaturen zwischen 50 und 300°C, vorzugsweise 120 - 250°C in Gegenwart von sauren Katalysatoren. Saure Katalysatoren im Sinne der Erfindung sind starke Säuren mit einem in Wasser gemessenen pk-Wert kleiner als 2, also starke Protonsäuren wie Chlorwasserstoff, Bromwasserstoff, Schwefelsäure, Sulfonsäure, p-Toluolsulfonsäure, Phosphorsäure, Phosphorige Säure, Trifluoressigsäure, ferner Lewissäuren wie Aluminiumchlorid, Zinkchlorid, Eisen-III-chlorid, Titantetrachlorid, Bortrifluorid, Antimonpentachhlorid und Addukte solcher Lewissäuren, wie $BF_3$-Etherat, $BF_3$-Hydrat, Ionenaustauscher auf der Basis von vernetzten sulfonierten Polystyrolen und säureaktivierte Tonerden auf der Basis von Bentonit und Montmorillonit.

Diese Katalysatoren werden in Mengen von 0,1 - 20 Gew.-%, vorzugsweise 0,2 - 10 Gew.-%, bezogen auf das Reaktionsgemisch, eingesetzt. Sie können nach Durchführung der Reaktion durch Neutralisation und Auswaschen oder durch Filtration entfernt werden. Die Reaktion kann in Gegenwart oder Abwesenheit von Verdünnungs- und Lösemitteln durchgeführt werden. Geeignete Lösemittel sind gegenüber den Reaktionspartnern inert und müssen leicht abzutrennen sein. Geeignet sind beispielsweise aliphatische und aromatische Kohlenwasserstoffe wie Dekalin, Benzin, Benzol, Toluol, Xylol, Cumol, Tetralin, Halogenaromaten wie Chlorbenzol, Dichlorbenzol, Brombenzol und Ether wie Dioxan und Anisol.

Die Durchführung der Reaktion erfolgt in der Regel so, daß man das aromatische Amin, das gegebenenfalls in

Lösung gebracht ist, nach Zugabe des Katalysators auf die Reaktionstemperatur bringt, sukzessive mit der alkylierenden Verbindung versetzt und dabei das gegebenenfalls sich abspaltende produkt wie Wasser oder Alkohol abdestilliert. Eine Aufarbeitung der Reaktionsprodukte ist häufig nicht erforderlich. Man kann jedoch, wie beschrieben, den Katalysator entfernen, flüchtige Bestandteile Abdestillieren und das Reaktionsprodukt als Sumpfprodukt, durch Ausfällen oder in besonderen Fällen durch Auskristallisieren aus geeigneten Lösungsmitteln isolieren.

Zur Herstellung der Polyurethane werden als Ausgangskomponenten eingesetzt:

Aliphatische, cycloaliphatische, araliphatische, aromatische und heterocyclische Polyisocyanate, wie sie z.B. von W. Siefken in Justus Liebigs Annalen der Chemie, 562, Seiten 75 bis 136, beschrieben werden, beispielsweise solche der Formel

$Q(NCO)_n$,

in der

n 2-4, vorzugsweise 2, und

Q einen aliphatischen Kohlenwasserstoffrest mit 2-18, vorzugsweise 6-10 C-Atomen,
einen cycloaliphatischen Kohlenwasserstoffrest mit 4-15, vorzugsweise 5-10 C-Atomen,
einen aromatischen Kohlenwasserstoffrest mit 6-15, vorzugsweise 6-13 C-Atomen,
oder einen araliphatischen Kohlenwasserstoffrest mit 8-15, vorzugsweise 8-13 C-Atomen,
bedeuten, z.B. 1,4-Tetramethylendiisocyanat, 1,6-Hexa-methylendiisocyanat, 1,12-Dodecandiisocyanat, Cyclobutan-1,3-diisocyanat, Cyclohexan-1,3- und -1,4-diiso-cyanat, 1-Isocyanato-3,3,5-trimethyl-5-isocyanato-methyl-cyclohexan, 2,4- und 2,6-Hexahydrotoluylendiiso-cyanat, Hexahydro-1,3- und/oder -1,4-phenyllendiiso-cyanat, Perhydro-2,4'- und/oder -4,4'-diphenylmethan-diisocyanat, sowie beliebige Stellungs- und Stereoisomeren-Gemische, 1,3- und 1,4-Phenylendiisocyanat, 2,4-und/oder 2,6-Toluylendiisocyanat, Diphenylmethan-2,4'-und/oder -4,4'-diisocyanat, sowie beliebige Gemische dieser Isomeren, ferner Naphthylen-1,5-diisocyanat.

Ferner kommen beispielsweise erfindungsgemäß in Frage: Triphenylmethan-4,4',4"-triisocyanat, Polyphenylpolymethylenpolyisocyanate, wie sie durch Anilin-Formaldehyd-Kondensation und anschließende Phosgenierung erhalten und z.B. in den GB-patentschriften 874 430 und 848 671 beschrieben werden, m- und p-Isocyanatophenylsulfonylisocyanate, perchlorierte Arylpolyisocyanate, Carbodiimidgruppen aufweisende polyisocyanate, Norbornan-Diisocyanate, Allophanatgruppen aufweisende Polyisocyanate, Isocyanuratgruppen aufweisende Polyisocyanate, Urethangruppen aufweisende Polyisocyanate, acylierte Harnstoffgruppen aufweisende Polyisocyanate, Biuretgruppen aufweisende Polyisocyanate, durch Telomerisationsreaktionen hergestellte Polyisocyanate, Estergruppen aufweisende Polyisocyanate, Umsetzungsprodukte der obengenannten Isocyanate mit Acetalen und polymere Fettsäureester enthaltende Polyisocyanate gemäß der US-Patentschrift 3 455 883.

Ferner ist es möglich, beliebige Mischungen der vorgenannten Polyisocyanate zu verwenden.

Eine ausführliche Aufzählung derartiger geeigneter Isocyanate und ihre Herstellungsweisen wird in der DE-OS 2 854 384 auf den Seiten 8-11 angeführt.

Besonders bevorzugt werden in der Regel die technisch leicht zugänglichen Polyisocyanate, z.B. das 2,4- und/oder 2,6-Toluylendiisocyanat ("TDI"), Polyphenyl-polymethylen-polyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate "modifizierte Polyisocyanate"), insbesondere solche modifizierten Polyisocyanate, die sich vom 2,4-und/oder 2,6-Toluylendiisocyanat bzw. vom 4,4'- und/ oder 2,4'-Diphenylmethandiisocyanat ableiten. Ferner sind symmetrische und unsymmetrische mit Methyl-, Ethyl- oder Isopropylgruppen substituierte Derivate der Diphenylmethan-diisocyanate geeignet.

Als höhermolekulare Polyol-Ausgangskomponenten werden Verbindungen mit mindestens zwei gegenüber Isocyanaten reaktionsfähigen Wasserstoffatomen von einem Molekulargewicht in der Regel von 400-10 000 eingesetzt. Hierunter versteht man neben Amino-, Thiol- oder Carboxylgruppen aufweisenden Verbindungen vorzugsweise Hydroxylgruppen aufweisende Verbindungen, insbesondere mit zwei bis acht Hydroxylgruppen, speziell solche von Molekulargewicht 600 bis 6000, vorzugsweise 800 bis 5000, z.B. mindestens zwei, in der Regel 2 bis 8, vorzugsweise aber 2 bis 4, Hydroxylgruppen aufweisende Polyester, Polyether, polythioether, Polyacetale, Polycarbonate und Polyesteramide, wie sie für die Herstellung von homogenen und von zellförmigen Polyurethanen an sich bekannt sind.

Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein.

Als Beispiele für solche Carbonsäuren und deren Derivate seien genannt: Adipinsäure, Sebacinsäure, phthalsäure(anhydrid), Isophthalsäure, Trimellitsäure, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure-(anhydrid), Fumarsäure, di- und trimerisierte ungesättigte Fettsäuren, Terephthalsäuredimethylester und Terephthalsäure-bis-glykolester. Als mehrwertige Alkohole kommen z.B. Ethylenglykol, Propylenglykol-(1,2) und -(1,3), Butylenglykol-(1,4) und -(2,3), Hexandiol-(1,6), Neopentylglykol,

1,4-Bis-hydroxymethylcyclohexan, 2-Methyl-1,3-propandiol, Glycerin, Trimethylolpropan, Hexantriol-(1,2,6), Butantriol-(1,2,4), Trimethylolethan, Pentaerythrit, Chinit, Mannit und Sorbit, Formit, Methylglykosid, ferner Di-, Tri-Tetraethylenglykol und höhere polyethylenglykole, Di-, Tri-, Tetrapropylenglykol und höhere Polypropylenglykole sowie Di-, Tri-, Tetrabutylenglykol und höhere Polybutylenglykole in Frage. Die Polyester können anteilig endständige Carboxylgruppen aufweisen. Auch Polyester aus Lactonen, z.B. ε-Caprolacton, oder aus Hydroxycarbonsäuren, z.B. ω-Hydroxycapronsäure, sind einsetzbar.

Auch die zur Anwendung kommenden, mindestens zwei, in der Regel zwei bis acht, vorzugsweise zwei bis drei, Hydroxylgruppen aufweisenden Polyether sind solche der an sich bekannten Art und werden z.B. durch Polymerisation von Tetrahydrofuran oder Epoxiden wie Ethylenoxid, Propylenoxid, Butylenoxid, Styroloxid oder Epichlorhydrin mit sich selbst oder durch Anlagerung dieser Epoxide, vorzugsweise von Ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z.B. Ethylenglykol, Propylenglykol-(1,3) oder -(1,2), Trimethylolpropan, Glycerin, Sorbit, 4,4'-Dihydroxy-di-phenylpropan, Anilin, Ethanolamin oder Ethylendiamin hergestellt. Auch Sucrosepolyether, sowie auf Formit oder Formose gestartete polyether kommen in Frage. Vielfach sind solche Polyether bevorzugt, die überwiegend (bis zu 90 Gew.-%, bezogen auf alle vorhandenen OH-Gruppen im Polyether) primäre OH-Gruppen aufweisen. Auch OH-Gruppen aufweisende Polybutadiene sind erfindungsgemäß geeignet.

Unter den Polythioethern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt, als Polyacetale z.B. die aus Glykolen, wie Diethylenglykol, Triethylenglykol, 4,4'-Dioxethoxydiphenyldimethylmethan, Hexandiol und Formaldehyd herstellbaren Verbindungen und Hydroxylgruppen aufweisende Polycarbonate solche der an sich bekannten Art, die z.B. durch Umsetzung von Diolen wie Propandiol-(1,3), Butandiol-(1,4) und/oder Hexan-diol-(1,6), mit Diarylcarbonaten, z.B. Diphenylcarbonat oder Phosgen hergestellt werden.

Zu den Polyesteramiden und Polyamiden zählen z.B. die aus mehrwertigen gesättigten oder ungesättigten Carbonsäuren bzw. deren Anhydriden und mehrwertigen gesättigten oder ungesättigten Aminoalkoholen, Diaminen, Polyaminen und deren Mischungen gewonnenen, vorwiegend linearen Kondensate.

Auch bereits Urethan- oder Harnstoffgruppen enthaltende Polyhydroxylverbindungen sowie gegebenenfalls modifizierte natürliche Polyole, wie Rizinusöl oder Kohlenhydrate, z.B. Stärke, sind verwendbar. Auch Anlagerungsprodukte von Alkylenoxiden an Phenol-Formaldehyd-Harze oder auch an Harnstoff-Formaldehydharze sind erfindungs-gemäß einsetzbar.

Die genannten Polyhydroxylverbindungen können vor ihrer Verwendung im Polyisocyanat-Polyadditionsverfahren noch in der verschiedensten Weise modifiziert werden: So läßt sich ein Gemisch aus verschiedenen Polyhydroxylverbindungen (z.B. aus einem Polyether- und einem Polyesterpolyol) durch Veretherung in Gegenwart einer starken Säure zu einem höhermolekularen Polyol kondensieren, welches aus über Etherbrücken verbundenen verschiedenen Segmenten aufgebaut ist. Es ist auch möglich, z.B. Triazingruppen einzuführen.

Von besonderem Interesse ist es in manchen Fällen, die höhermolekularen Polyhydroxylverbindungen durch Reaktion mit Isatosäureanhydrid vollständig oder teilweise in die entsprechenden Anthranilsäureester mit endständigen aromatischen Aminogruppen überzuführen.

Auch durch Umsetzung von NCO-Präpolymeren mit Hydroxylgruppen aufweisenden Enaminen, Aldiminen oder Ketiminen und anschließende Hydrolyse erhält man höhermolekulare endständige Aminogruppen aufweisende Verbindungen. Weitere Herstellungsverfahren für höhermolekulare Verbindungen mit endständigen Aminogruppen oder Hydrazidgruppen werden in der DE-OS 1 694 152 (US-PS 3 625 871) beschrieben.

Erfindungsgemäß können gegebenenfalls auch polyhydroxylverbindungen eingesetzt werden, in welchen hochmolekulare Polyaddukte bzw. polykondensate oder polymerisate in feindisperser oder gelöster Form enthalten sind. Derartige Polyhydroxylverbindungen werden z.B. erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) in situ in den obengenannten Hydroxylgruppen aufweisenden Verbindungen ablaufen läßt. Es ist aber auch möglich, eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z.B. durch Polymerisation von Styrol und Acrylnitril in Gegenwart von Polyethern erhalten werden, sind für das erfindungsgemäße Verfahren geeignet. Bei Verwendung von Polyetherpolyolen, welche durch Pfropfpolymerisation mit Vinylphosphonsäureestern sowie gegebenenfalls (Meth)acrylnitril, (Meth)acrylämid oder OH-funktionellen (Meth)acrylsäureestern modifiziert wurden, erhält man Kunststoffe von besonderer Flammwidrigkeit.

Bei der Verwendung von modifizierten Polyhydroxylverbindungen der obengenannten Art als Ausgangskomponente im Polyisocyanat-Polyadditionsverfahren entstehen in vielen Fällen polyurethankunststoffe mit wesentlich verbesserten mechanischen Eigenschaften.

Vertreter der genannten erfindungsgemäß zu verwendenden Verbindungen sind z.B. in High Polymers, Vol. XVI, "Polyurethanes, Chemistry and Technology", verfaßt von Saunders-Frisch, Interscience Publishers, New York, London, Band I, 1962, Seiten 32-42 und Seiten 44-54 und Band II, 1964, Seiten 5-6 und 198-199, ferner im Kunststoff-Handbuch, Band VII, Vieweg-Höchtlen, Carl-Hanser-Verlag, München, 1966, z.B. auf den Seiten 45-71 sowie besonders in der DE-OS 2 854 834, Seiten 11-21 beschrieben. Selbstverständlich können Mischungen der

obengenannten Verbindungen mit mindestens zwei gegenüber Isocyanaten reaktionsfähigen Wasserstoffatomen mit einem Molekulargewicht von 400 - 10 000, z.B. Mischungen von Polyethern und Polyestern, eingesetzt werden.

Gegebenenfalls können als Ausgangskomponenten auch relativ niedermolekulare Verbindungen mit mindestens zwei gegenüber Isocyanaten reaktionsfähigen Wasserstoffatomen und einem Molekulargewicht von 32 bis 400, verwendet werden. Auch hier versteht man hierunter Hydroxyl- und/oder Amino- und/oder Thiol- und/oder Carboxylgruppen aufweisende Verbindungen, vorzugsweise Hydroxyl- und/oder Aminogiuppen aufweisende Verbindungen, die als Kettenverlängerungsmittel oder Vernetzungsmittel dienen. Diese auch in Mischung einsetzbaren Verbindungen weisen in der Regel 2 bis 8, vorzugsweise 2 bis 4, gegenüber Isocyanaten reaktionsfähige Wasserstoffatome auf. Als Beispiele für derartige Verbindungen seien genannt: Ethylenglykol, Propylenglykol-(1,2) und -(1,3), Butylen-glykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Dibrombutendiol, Glyzerin, Trimethylolpropan, Hexantriol-(1,2,6), Trimethylolethan, Pentaerythrit, Chinit, Mannit und Sorbit, Ricinusöl, Di-, Tri-, Tetra- und höhere Polyethylenglykole, Di-, Tri-, Tetrapropylenglykol und höhere Polypropylenglykole, Di-, Tri- und Tetrabutylenglykol, höhere Polybutylenglykole jeweils mit einem Molekulargewicht bis 400, 4,4'-Dihydroxydiphenylpropan, Dihydroxymethyl-hydrochinon, Ethanolamin, Diethanolamin, N-Methyldiethanolamin, Triethanolamin, 3-Aminopropanol.

Als niedermolekulare Polyole kommen erfindungsgemäß auch die Gemische von Hydroxyaldehyden und Hydroxyketonen ("Formose") bzw. die hieraus durch Reduktion erhaltenen mehrwertigen Alkohole ("Formit") in Frage, mit Vorteil in Kombination mit Aminoplastbildnern und/oder Phosphiten ein. Auch Lösungen von Polyisocyanatpolyadditionsprodukten, insbesondere von ionische Gruppen aufweisenden Polyurethanharnstoffen und/oder von Polyhydrazodicarbonamiden, in niedermolekularen, mehrwertigen Alkoholen kommen erfindungsgemäß als Polyolkomponente in Betracht.

Erfindungsgemäß geeignete aliphatische Diamine sind beispielsweise Ethylendiamin, 1,4-Tetramethylendiamin, 1,11-Undecamethylendiamin, 1,12-Docecamethylendiamin, p-Xylylendiamin, Bis-(3-aminopropyl)methylamin, sowie deren Gemische; cycloaliphatische Diamine wie 1-Amino-3,3,5-trimethyl-5-aminomethylcyclohexan ("Isophorondiamin"), 2,4- und/oder 2,6-Hexahydrotoluylendiamin, Perhydro-2,4'- und/oder 4,4'-diaminodiphenylmethan, Diamino-perhydroanthrazene oder ihre Gemische von Stellungs- und/oder Stereoisomeren; ferner cycloaliphatische Triamine gemäß DE-Offenlegungsschrift 2 614 244. Auch Hydrazin und substituierte Hydrazine, sowie Säuredihydrazide kommen erfindungsgemäß in Betracht, z.B. Carbodihydrazid, Oxalsäuredihydrazid, die Dihydrazide von Malonsäure, Adipinsäure, ß-Methyladipinsäure, Hydracrylsäure und Terephthalsäure; Semicarbazido-alkylen-hydrazide wie z.B. ß-Semicarbazidopropionsäurehydrazid, Semicarbazidoalkylencarbazinester wie z.B. 2-Semicarbazidoethylcarbazinester oder auch Amino-semicarbazid-Verbindungen wie z.B. ß-Aminoethyl-semicarbazido-carbonat. Zur Steuerung ihrer Reaktivität können die Aminogruppen ganz oder teilweise durch Aldimin- bzw. Ketimin-Gruppen blockiert sein.

Als Beispiele für aromatische Diamine seien Bisanthranilsäureester, 3,5- und 2,4-Diaminobenzoesäureester, die in den DE-Offenlegungsschriften 1 803 635, 2 040 650 und 2 160 589 beschriebenen estergruppenhaltigen Diamine, die Ethergruppen aufweisenden Diamine gemäß DE-Offenlegungsschriften 1 770 525 und 1 809 172, gegebenenfalls in 5-Stellung substituierte 2-Halogen-1,3-Phenylen-diamine, 3,3'-Dichlor-4,4'-diaminodiphenylmethan, Toluylendiamin 4,4'-Diaminodiphenylmethan, 4,4'-Di-aminodiphenyldisulfide, Diaminodiphenyldithioether, durch Alkylthiogruppen substituierte aromatische Diamine, Diaminobenzolphosphonsäureester, Sulfonatoder Carboxylatgruppen enthaltende aromatische Diamine sowie die in der DE-Offenlegungsschrift 2 635 400 aufgeführten hochschmelzenden Diamine genannt. Beispiele für aliphatisch-aromatische Diamine sind die Aminoalkylthioaniline.

Als Kettenverlängerungsmittel können erfindungsgemäß auch Verbindungen wie 1-Mercapto-3-aminopropan, gegebenenfalls substituierte Aminosäuren, z.B. Glycin, Alanin, Valin, Serin und Lysin sowie gegebenenfalls substituierte Dicarbonsäuren, beispielsweise Bernsteinsäure, Adipinsäure, Phthalsäure, 4-Hydroxy-phthalsäure und 4;Aminophthalsäure verwendet werden.

Ferner können gegenüber Isocyanaten monofunktionelle Verbindungen in Anteilen von 0,01 bis 10 Gew.-%, bezogen auf Polyurethanfeststoff, als sogenannte Kettenabbrecher mitverwendet werden. Derartige monofunktionelle Verbindungen sind z.B. Monoamine wie Butyl- und Dibutylamin, Stearylamin, N-Methylstearylamin, Pyrrolidin, und Cyclohexylamin, Monoalkohole wie Butanol, 2-Ethyl-hexanol, Cyclohexanol, Ethylenglykolmonoethylether.

Weitere erfindungsgemäß geeignete niedermolekulare Polyole vom Molekulargewicht bis 400 sind Esterdiole, z.B. δ -Hydroxybutyl- ε -hydroxy-capronsäureester, ω-Hydroxyhexyl- γ-hydroxybuttersäureester, Adipin-säure-bis-(ß-hydroxyethyl)ester und Terephthalsäure-bis(ß-hydroxyethyl)ester; Diolurethane z.B. 1,6-Hexa-methylen-bis-(ß-hydroxyethylurethan) oder 4,4'-Di-phenylmethan-bis-(δ-hydroxybutylurethan); sowie Diolharnstoffe z.B. 4,4'-Diphenylmethan-bis-(ß-hydroxy-ethylharnstoff) oder die Verbindung

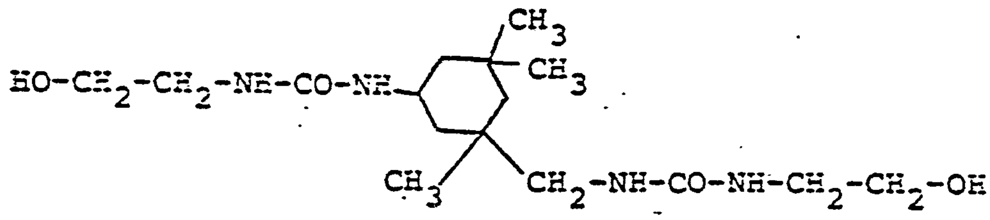

Für manche Zwecke ist es vorteilhaft, Polyole einzusetzen, welche Sulfonat- und/oder Phosphonatgruppen enthalten (DE-Offenlegungsschrift 2 719 372), vorzugsweise das Addukt von Bisulfit an Butandiol-1,4 bzw. dessen Alkoxylierungsprodukte.

Diese niedermolekularen Verbindungen mit Molekulargewichten von 32 bis 400 werden ausführlich in der DE-OS 2 854 384, z.B. auf den Seiten 20 bis 26 beschrieben und es werden dort weitere Beispiele genannt.

Katalysatoren der an sich bekannten Art können mitverwendet werden, z.B. tert.-Amine wie Triethylamin, N-Methylmorpholin, Tetramethylethylendiamin, 1,4-Diazabicyclo-(2,2,2)-octan, Bis-(dimethylaminoalkyl)piperazine, Dimethylbenzylamin, 1,2-Dimethylimidazol, mono- und bicyclische Amidine, Bis-(dialkylaminoalkvlether), sowie Amid- (vorzugsweise Formamid)-Gruppen aufweisenden tert.-Amine. Als Katalysatoren kommen auch an sich bekannte Mannichbasen aus sekundären Aminen und Aldehyden oder Ketonen in Frage. Erfindungsgemäß werden besonders organische Metallverbindungen wie organische Zinnverbindungen als Katalysatoren verwendet. Als organische Zinnverbindungen kommen neben schwefelhaltigen Verbindungen wie Di-n-octylzinn-mercaptid vorzugsweise Zinn-(II)-Salze von Carbonsäuren wie Zinn-(II)-Acetat, Zinn-(II)-Ethylhexoat und die Zinn(IV)-Verbindungen, z.B. Dibutylzinndichlorid, Dibutylzinndiacetat, Dibutylzinndilaurat oder Dibutylzinnmaleat in Betracht. Selbstverständlich können alle Katalysatoren als Gemische eingesetzt werden. Weitere Vertreter von verwendbaren Katalysatoren sowie Einzelheiten über die Wirkungsweise sind im Kunststoffhandbuch Band VII, herausgegeben von Vieweg und Höchtlen, Carl-Hanser-Verlag, München 1966, z.B. auf den Seiten 96 bis 102 beschrieben bzw. in der DE-OS 2 854 384 aufgeführt. Als Hilfs- und Zusatzmittel können verwendet werden:

Anorganische oder organische Substanzen als Treibmittel, insbesondere Verbindungen wie Methylenchlorid, Chloroform, Vinylidenchlorid, Monofluortrichlormethan, Chlordichlordifluormethan, ferner Luft, $CO_2$ oder Stickoxid. Weitere Beispiele für Treibmittel sowie Einzelheiten über deren Verwendung sind im Kunststoffhandbuch, herausgegeben von Vieweg und Höchtlen, Carl-Hanser-Verlag München 1966, z.B. auf den Seiten 108 und 109, 453 bis 455 und 507 bis 510 beschrieben.

Oberflächenaktive Zusatzstoffe, wie Emulgatoren und Schaumstoffinitiatoren werden in üblicher Weise mitverwendet. Als Emulgatoren kommen z.B. Natriumsalze von Rizinusöl-Sulfonaten oder Salze von Fettsäuren mit Aminen wie ölsaures Diethylamin, ferner Alkali- oder Anmoniumsalze von Sulfonsäuren wie Dodecylbenzolsulfonsäure oder Dinaphthylmethan-disulfonsäure in Frage.

Als Schaumstabilisatoren kommen vor allem Polyethersiloxane, speziell wasserlösliche Vertreter, in Frage. Reaktionsverzögerer, z.B. sauer reagierende Stoffe wie Salzsäure, Chloressigsäure oder organische Säurehalogenide, ferner Zellregler der an sich bekannten Art wie Paraffine oder Fettalkohole oder Dimethylpolysiloxane, sowie Pigmente oder Farbstoffe und/oder Flammschutzmittel der an sich bekannten Art, ferner Stabilisatoren gegen Alterungs- und Witterungseinflüsse, Weichmacher, fungistatisch und/oder bakteriostatisch wirkende Substanzen, sowie Füllstoffe können mitverwendet werden. Einzelheiten zu diesem Zusatzund Hilfsstoffen können der DE-OS 2 854 384 auf Seiten 26 bis 31 und den dort zitierten Literaturstellen entnommen werden, ebenso die Möglichkeiten zur Schaumstoffherstellung.

Die Schaumstoffherstellung kann sowohl als Freischaum als auch als Formschaum in der üblichen Weise erfolgen. Selbstverständlich können die Schaumstoffe auch zur Blockverschäumung oder nach an sich bekannten Doppeltransportbandverfahren oder irgendeiner weiteren Variante der Schaumstofftechnik hergestellt werden.

**Herstellungsbeispiele für die Stabilisatoren**

**Beispiel 1**

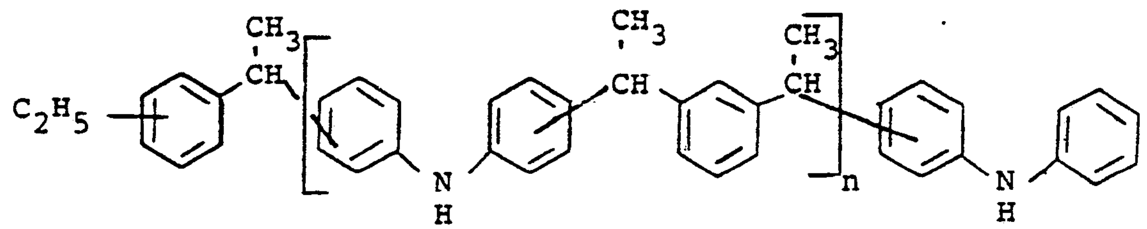

507 g (3 Mol) Diphenylamin und 10 g säureaktivierte Tonerde (Tonsil® Optimum) werden unter Rühren und Stickstoff auf 145-150° aufgeheizt und in 3 h tropfenweise mit 217 g technischem Divinylbenzol (bestehend aus 61 % m- und p-Divinylbenzol und 39 % Ethylvinylbenzol) versetzt und noch 1 h bei 150° gehalten. Noch heiß wird unter Stickstoff abgedrückt, der Filterkuchen mit heißem Xylol gewaschen und abgedrückt. Die vereinigten Filtrate werden eingedampft und bis zu einer Sumpftemperatur von 190° bei 14 mbar von flüchtigen Bestandteilen befreit. Man erhält die Titelverbindung als hellbraunes hochviskoses Harz (620 g).

Nach gelchromatographischer Untersuchung beträgt das Molgewicht ca. 1800.

**Beispiel 2**

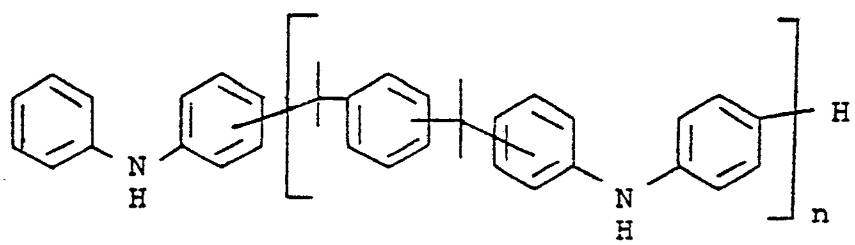

Ein Gemisch von 338 g (2 Mol) Diphenylamin, 194 g (1 Mol) α,α'-Dihydroxy-m-/p-diisopropylbenzol (Molverhältnis 3:2) und 10 g säureaktivierter Tonerde wird aufgeheizt unter Stickstoff und Rühren. Ab 120-125° Sumpftemperatur geht azeotrop Wasser über, das bei laufend steigender Temperatur abdestilliert wird. Schließlich hält man noch 3 h bei 180°. Nach Abkühlen auf 100° wird mit Toluol verdünnt und heiß abgedrückt. Nach Waschen des Filterkuchens und Eindampfen der vereinigten Filtrate bis zu einer Sumpftemperatur von 190° bei 15 mbar verbleiben 480 g der Titelverbindung als braunes Weichharz.

**Beispiel 3**

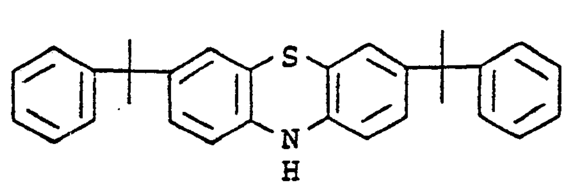

1000 g Phenothiazin, 1000 ml Xylol und 100 g säureaktivierte Tonerde werden unter Rühren und Stickstoff aufgeheizt auf 180°, wobei ein Teil des Xylols abdestilliert wird. Innerhalb von 3 h tropft man nun 1190 g α-Methylstyrol zu und hält noch 15-30 min bei 180°. Nach Verdünnen mit 2 1 Xylol wird heiß abgedrückt, mit Xylol nachgespült und das noch heiße klare Filtrat mit 2,5 l Ligroin verdünnt und unter Rühren und Stickstoff abkühlen lassen. Nach Absaugen, Waschen mit Xylol/Ligroin und Trocknen erhält man 1750 g der Titelverbindung als hellgelbe Kristalle, Fp. 129-133°.

**Beispiel 4**

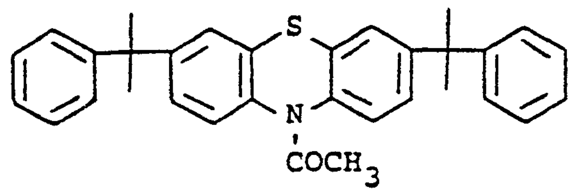

63 g (0,14 Mol) der Verbindung aus Beispiel 3 werden in 150 ml Xylol mit 29 g (0,29 Mol) Acetanhydrid 1 h unter Rückfluß gekocht und anschließend eingedampft bis zu einer Sumpftemperatur von 120° bei 13 mbar. Man erhält die Titelverbindung als sprödes Harz, dessen NMR-Spektrum und Elementaranalyse mit der angegebenen Struktur übereinstimmt.

**Beispiel 5**

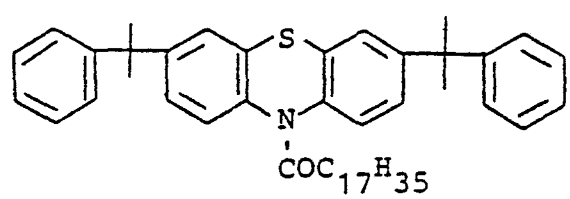

87 g (0,2 Mol) der Verbindung aus Beispiel 3 und 63 g (0,2 Mol) Stearinsäurechlorid werden langsam aufgeheizt unter Rühren und Stickstoff, wobei ab 90-100° Chlorwasserstoff entwickelt wird. Nach 3-4 h bei 100-120° ist die Hauptreaktion beendet. Zur Entfernung von Chlorwasserstoff wird noch 2 h bei 100-120° Stickstoff durchgeleitet. Man erhält 138 g der Titelverbindung als hellbraunes Harz.

**Beispiel 6**

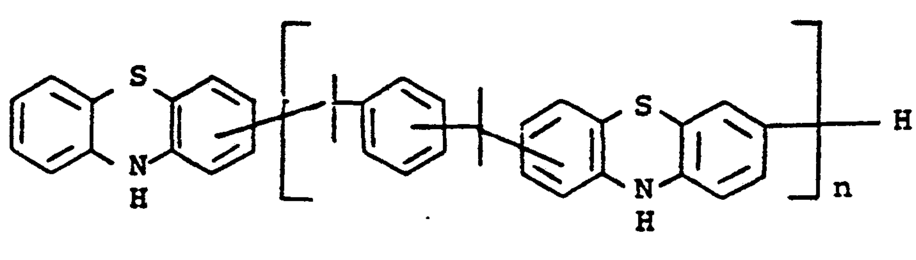

100 g (0,5 Mol) Phenothiazin werden unter Rühren, Rück-fluß und Stickstoff in 200 ml Xylol gelöst. Man gibt 5 g säureaktivierte Tonerde hinzu und destilliert soviel Xylol ab, daß die Sumpftemperatur 160-170° beträgt. Portionsweise werden nun 97 g (0,5 Mol)α,α'-Dihydroxy-m/p-diisopropylbenzol (Molverhältnis 3/2) eingetragen und azeotrop Wasser ausgekreist. Wenn kein Wasser mehr übergeht, hält man noch ca. 3 h bei 160°, filtriert über eine Drucknutsche und dampft das Filtrat ein bis zu einer Sumpftemperatur von 190° bei 30 mbar. Man erhält ein braunes sprödes Harz (171 g).

**Beispiel 7**

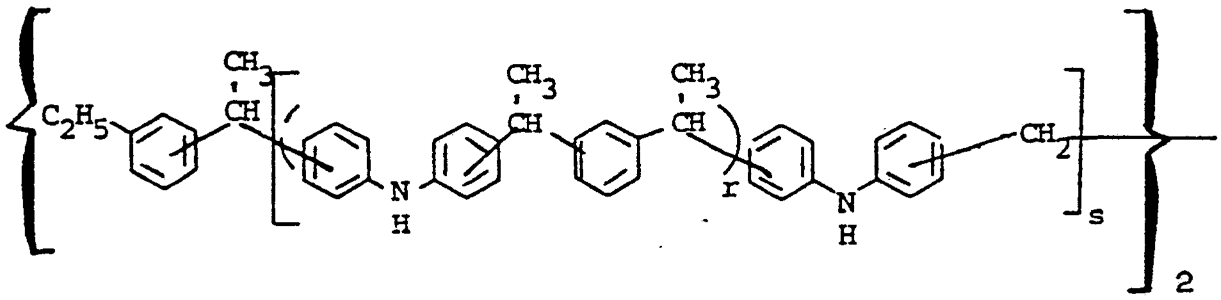

100 g der Verbindung aus Beispiel 1 werden unter Stickstoff und Rühren in Isopropanol gelöst, mit 3 ml konzentrierter Salzsäure rückfließend erhitzt und 12 ml 35 %iges Formalin zugetropft. Der ausfallende Niederschlag wird durch Zugabe von Xylol in Lösung gebracht. Man kocht noch 2 h unter Rückfluß, trennt die Phasen, wäscht die organische mit überschüssiger verdünnter Natronlauge, 3 x mit Wasser, filtriert und dampft die organische Phase bis zu einer Sumpftemperatur von 160° bei 20 mbar ein. Man erhält ein hochviskoses braunes Harz, das beim Abkühlen spröde wird (102 g).

**Beispiel 8**

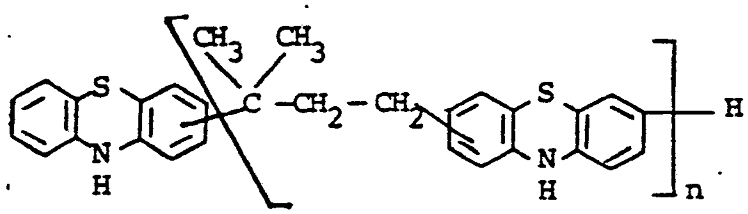

Ein Gemisch von 199 g (1 Mol) Phenothiazin und 100 ml Xylol werden unter Stickstoff und Rühren auf 150-160° gebracht und die klare Lösung mit 20 g säureaktivierter Tonerde (Tonsil Optimum) versetzt. In 4 h werden nun 68 g (1 Mol) Isopren aus einem gekühlten Tropftrichter zudosiert. Man hält noch 1 h bei 150-160°, verdünnt evtl. mit Xylol und filtriert durch eine Drucknutsche. Das Filtrat liefert nach Eindampfen bis 190° im Sumpf und 18 mbar 250 g eines braunen Harzes.

**Beispiel 9**

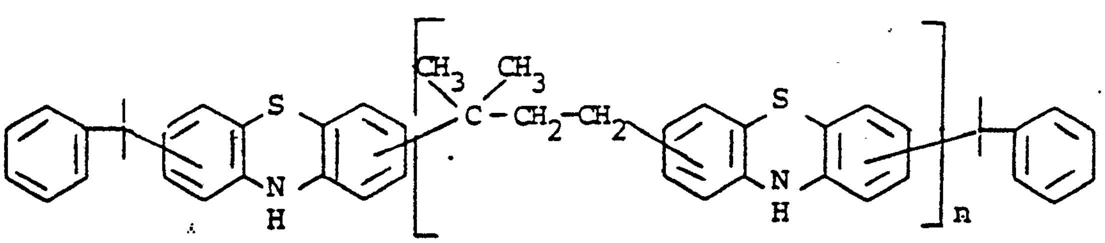

100 g der Verbindung aus Beispiel 8 in 100 ml Xylol werden unter Stickstoff und Rühren in Gegenwart von 2 g säureaktivierter Tonerde bei 150° in 4 h mit 10 g α-Methylstyrol umgesetzt. Nach Filtrieren und Eindampfen verbleiben 110 g eines braunen spröden Harzes.

**Beispiel 10**

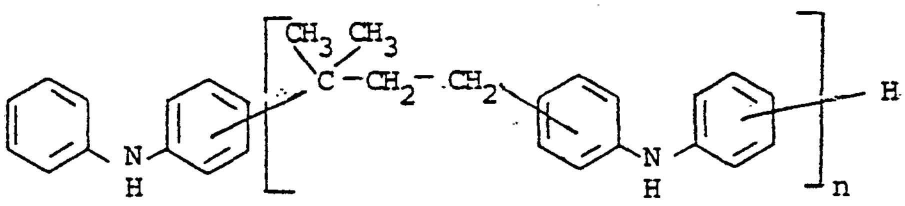

169 g (1 Mol) Diphenylamin und 20 g säureaktivierte Tonerde werden unter Rühren und Stickstoff auf 180° gebracht und während 2-3 h mit 68 g (1 Mol) Isopren aus einem gekühlten Tropftrichter versetzt. Man hält noch 2 h bei 180°, verdünnt mit Xylol, filtriert und dampft ein bis 150° im Sumpf bei 30 mbar. Man erhält 195 g eines braunen Harzes. (Molgewicht ca. 3200 (gelchromatographisch bestimmt)).

**Beispiel 11**

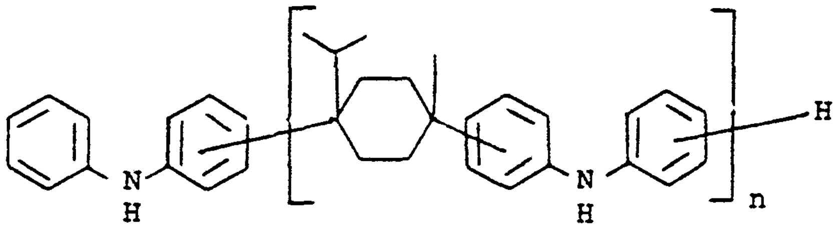

Zu einem Gemisch von 169 g (1 Mol) Diphenylamin und 20 g säureaktivierter Tonerde werden bei 175-180° in 2 h unter Rühren und Stickstoff 136 g (1 Mol) Limonen zugetropft. Man hält noch 2 h bei 190°, verdünnt mit Xylol, filtriert und dampft ein bis 190° im Sumpf bei 10 mbar. Es verbleiben 302 eines schwach gelben Harzes.

**Beispiel 12**

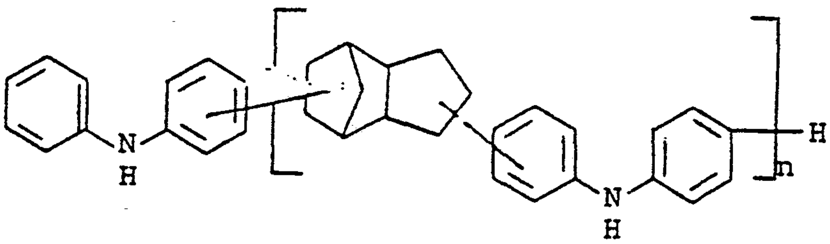

Beispiel 11 wird wiederhoit, aber statt 136 g Limonen werden 132 g (1 Mol) Dicyclopentadien eingesetzt. Man erhält 295 g eines braunen Harzes.

**Beispiel 13**

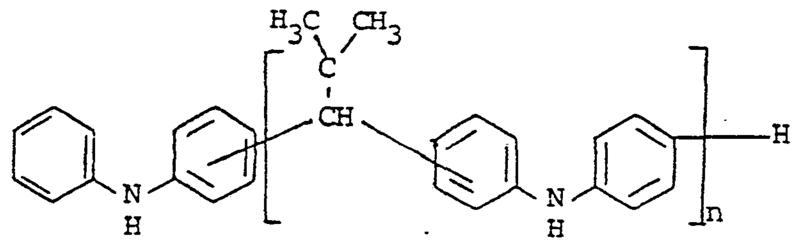

Ein Gemisch von 169 g (1 Mol) Diphenylamin und 20 g (0,2 Mol) konzentrierter Salzsäure wird auf 190-200° erhitzt, wobei Wasser abdestilliert. Zu der heißen Schmelze tropft man in 3-4 h unter Wasserabscheidung Isobutyraldehyd zu, bis 70-75 g umgesetzt sind. Man hält noch 1 h bei 200-210°, kühlt ab, löst in Toluol, stellt mit Ammoniaklösung alkalisch, trennt die organische Phase ab, wäscht mit $H_20$ und dampft im Wasserstrahlvakuum bis zu einer Sumpftemperatur von 210°/17 mbar ein. Es bleibt ein braunes Harz (160 g).

**Beispiel 14**

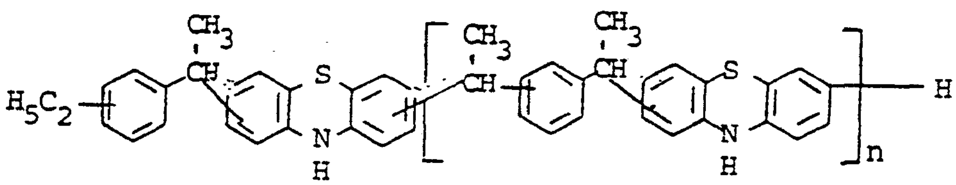

199 g (1 Mol) Phenothiazin und 20 g säureaktivierte Tonerde werden mit 100 ml Xylol unter Stickstoff auf 160-170° gebracht, wobei etwas Xylol abdestilliert wird. Unter Rühren tropft man 73 g technisches Divinylbenzol (61 % m/p-Divinylbenzol, 39 % Ethylvinylbenzol) in 2 h zu, hält noch 1 h bei 170°, filtriert und dampft ein. Es hinterbleiben 265 g eines braunen Harzes mit Molekulargewichten bis zu 2700 (laut Gelchromatographie).

**Beispiel 15**

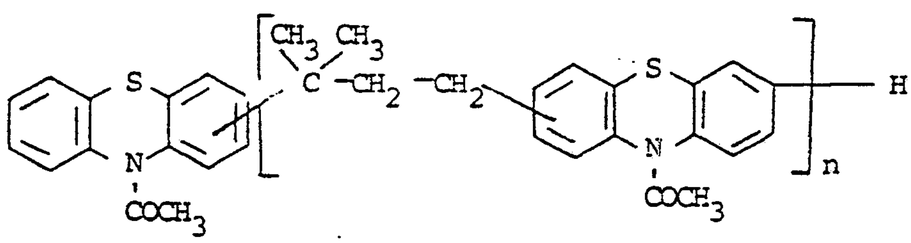

100 g (0 44 Mol) der Verbindung aus Beispiel 8 werden unter Rühren und Stickstoff mit 87 g (0,88 Mol) Acetanhydrid bei 134-155° umgesetzt, wobei über eine kurze Vigreuxkolonne Essigsäure abdestilliert wird. Überschüssiges Acetanhydrid wird zum Schluß im Wasserstrahlvakuum bei 20 mbar bei 170° Sumpftemperatur abdestilliert. Man erhält 118 g eines grünlichbraunen glasigen Harzes, dessen NMR-Spektrum mit der Titelverbindung im Einklang steht.

**Beispiel 16**

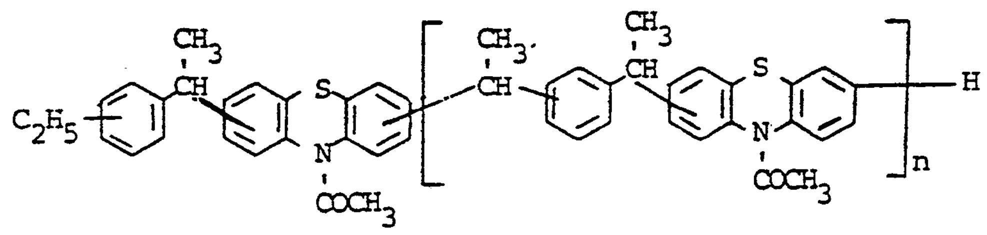

100 g der Verbindung aus Beispiel 14 werden wie in Beispiel 15 beschrieben mit 76 g Acetanhydrid umgesetzt. Man erhält 117 g glasiges grünliches Harz.

**Beispiel 17**

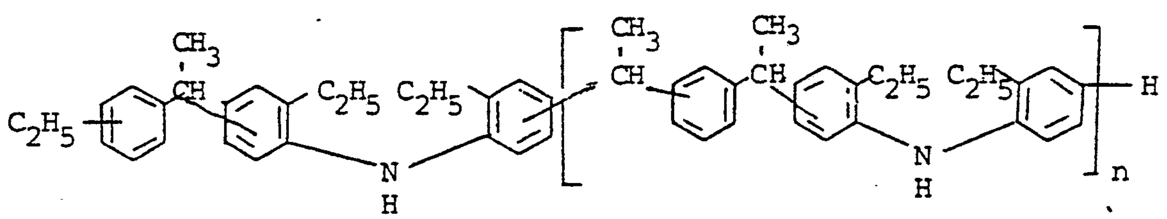

113 g 2,2'-Diethyldiphenylamin und 5 g säureaktivierte Tonerde (Tonsil Optimum) werden unter Stickstoff und Rühren auf 140-145° gebracht und tropfenweise mit 81 g technischem Divinylbenzol (siehe Eeispiel1) in 1 h versetzt. Man hält 2,5 h bei 140°, verdünnt mit Xylol, filtriert und dampft ein. Man erhält ein schwachbraunes Harz (165 g).

**Beispiel 18**

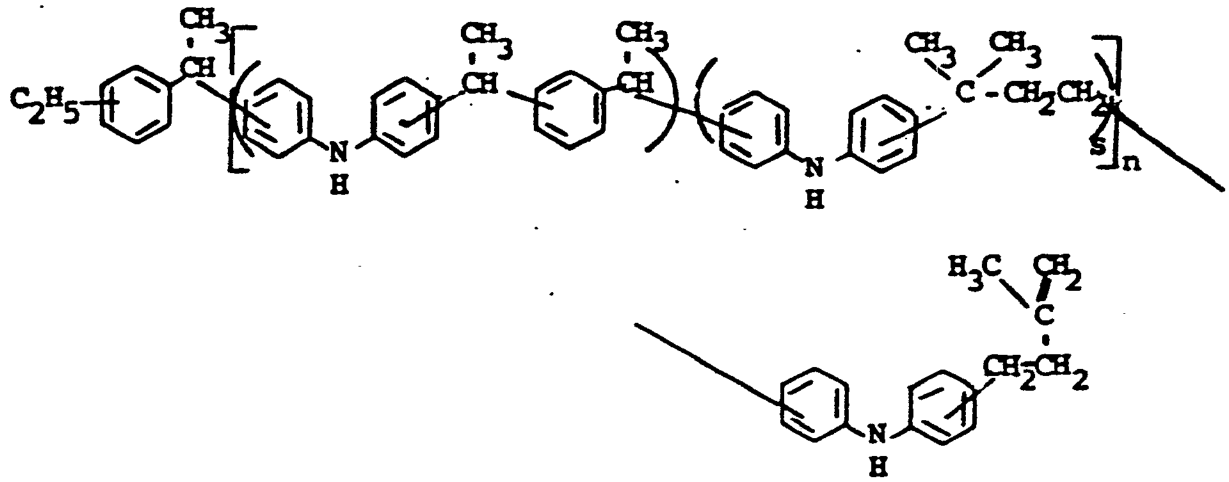

Zu 100g des produktes aus Beispiel 1 und 3 g säureaktivierter Tonerde in 100 g o-Dichlorbenzol werden unter Rühren und Stickstoff bei 180° 10 g Isopren mit Unterbrechungen über 4 h zugetropft. Nach weiteren 30 Minuten wird filtriert und bis 180°/10 mbar im Sumpf eingedampft. Es bleiben 108 g hellbraunes Harz. Molekulargewicht bis ca. 2500 (Gelchromatographie).

**Beispiel 19**

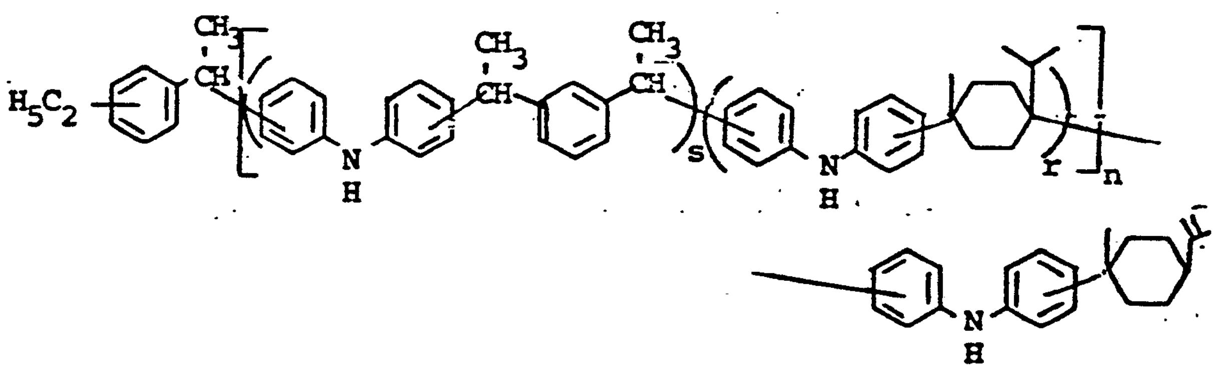

Beispiel 18 wird wiederholt, aber statt Isopren werden 30 g Limonen in 1 h zugetropft, noch 2 h bei 180° gerührt. Nach Aufarbeitung erhält man 120 g sprödes braunes Harz. Molekulargewicht bis etwa 2500 (Gelchromatographie).

**Beispiel 20**

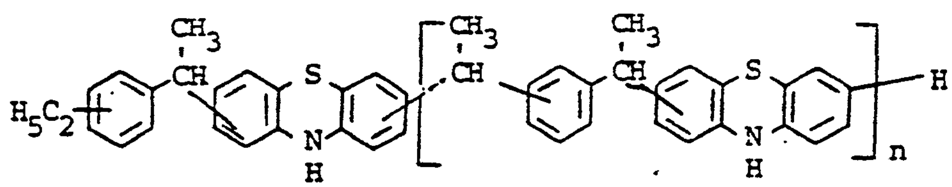

Zu einer Mischung von 199 g (1 Mol) Phenothiazin und 200 ml Xylol gibt man unter $N_2$ und Rühren 26 g konzentrierte Salzsäure, destilliert azeotrop Wasser und dann Xylol ab, bis die Sumpftemperatur 160° beträgt. Zu dieser Schmelze tropft man in 3,5 h 164 g techniches Divinylbenzol, hält noch 2 h bei 160°, nimmt in Xylol auf, wäscht bei 80-90° mit verdünnter Natronlauge bis zur basischen Reaktion, dann mit Wasser. Die organische Phase wird bis zu 170°/25 mbar im Sumpf eingedampft. Man erhält 365 g eines gelbbraunen glasigen Harzes.

**Beispiel 21**

Beispiel 20 wird wiederholt mit 5 g statt 26 g konzentrierter Salzsäure. Die Ausbeute beträgt 315 g eines hellbraunen Harzes.

**Beispiel 22**

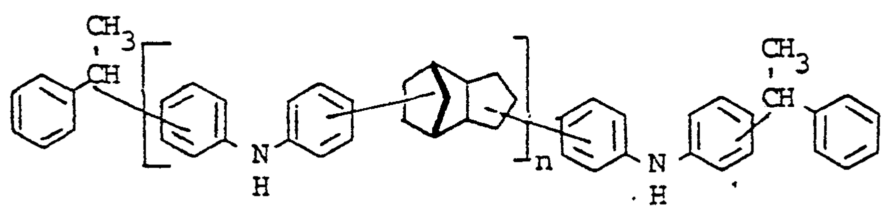

100 g des Produktes aus Beispiel 12 und 5 g säureaktivierte Tonerde werden bei 150° unter Rühren und Stickstoff in 30 min mit 50 g Styrol tropfenweise versetzt und noch 3 h bei 150° gehalten. Nach Filtrieren und Eindampfen bis 170°/20 mbar im Sumpf bleiben 124 g eines hellbraunen Harzes.

**Beispiel 23**

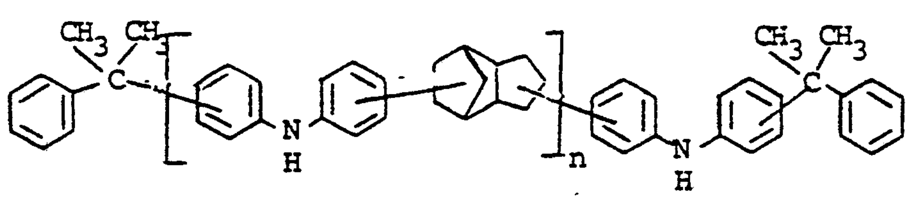

Beispiel 22 wird wiederholt, aber statt Styrol werden 50 g α-Methylstyrol eingesetzt. Man erhält 118 g eines hellbraunen spröden Harzes.

**Beispiel 24**

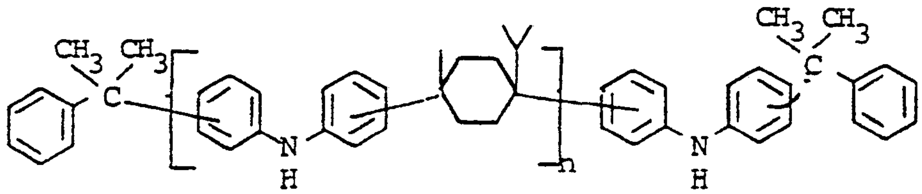

100 g des Produktes aus Beispiel 11 werden mit 5 g säureaktivierter Tonerde unter Rühren und Stickstoff auf 150° gebracht und dazu in 30 min 50 g α-Methyl-styrol getropft. Nach weiteren 2-3 h bei 150° wird heiß durch eine Drucknutsche filtriert und bis 190°/ 15 mbar im Sumpf eingedampft. Man erhält 135 g eines gelben spröden Harzes.

**Beispiel 25**

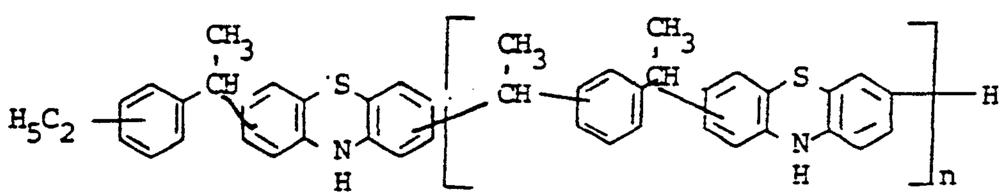

Zu einer Schmelze von 199 g (1 Mol) Phenothiazin mit 2 g säureaktivierter Tonerde werden bei 150° unter Rühren und Stickstoff in 3 h 164 g technisches Divinylbenzol (siehe Beispiel 1) zugetropft, das Reaktionsgemisch noch 4 h bei 150-160° gehalten, mit Xylol verdünnt, heiß filtriert und bis 180°/16 mbar im Sumpf eingedampft. 320 g eines gelbbraunen glasigen Harzes.

**Beispiel 26**

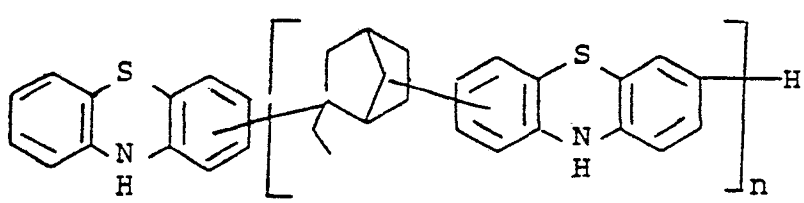

Eine Schmelze von 199 g (1 Mol) phenothiazin mit 10 g säureaktivierter Tonerde wird bei 180° in 3 h unter Stickstoff und Rühren mit 120 g (1 Mol) Ethylennorbornen tropfenweise versetzt und nach 2 h bei 180° gerührt. Nach Abdrücken und Eindampfen bis 180°/20 mbar bleiben 300 g eines braunen Harzes.

**Beispiel 27**

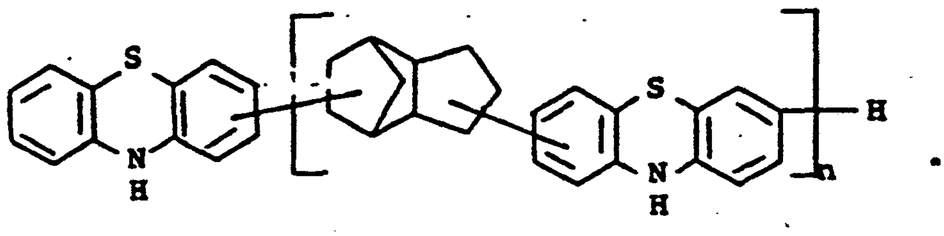

Zu einer Schmelze von 199 g (1 Mol) phenothiazin, die 10 g säureaktivierte Tonerde enthält, tropft man unter Rühren und Stickstoff 132 g (1 Mol) Dicyclopentadien in 2 h bei 160-170° zu und hält dann noch 2-3 h unter den Reaktionsbedingungen. Nach Abdrücken und Eindampfen bis 190°/0,6 mbar bleiben 150 g eines braunen spröden Harzes.

**Beispiel 28**

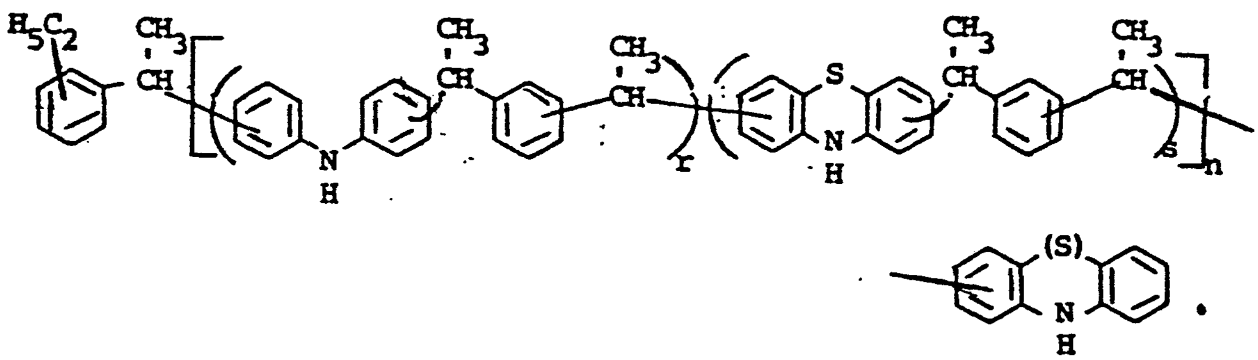

Ein Gemisch von 84,5 g (0,5 Mol) Diphenylamin, 99,5 g (0,9 Mol) phenothiazin und 10 g säureaktivierter Tonerde wird auf 150° unter Stickstoff und Rühren erhitzt und in 3 h mit 73 g technischem Divinylbenzol (siehe Beispiel 1) tropfenweise versetzt. Nach einer weiteren Stunde bei 150° wird in Xylol aufgenommen, heiß abgedrückt und bis zu einer Sumpftemperatur von 150°/10 mbar eingedampft. 238 g eines grünbraunen spröden Harzes.

**Beispiel 29**

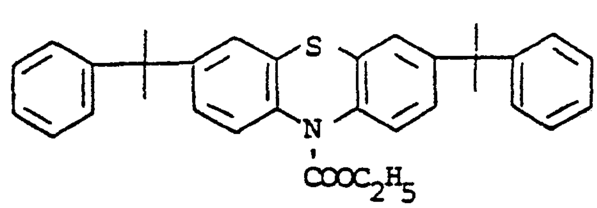

90 g der Verbindung aus Beispiel 3 und 22 g Chlorameisensäureethylester werden unter Rühren und Stickstoff langsam aufgeheizt, bis bei 100-110° lebhaft Chlorwasserstoff entwickelt wird. Nach ca. 1 h hört die Gasentwicklung fast auf, man erhitzt daher auf 120° und leitet Stickstoff durch die Mischung, um den restlichen Chlorwasserstoff zu entfernen. Man erhält 103 g eines hellgrüngrauen, klaren spröden Harzes.

**Beispiel 30**

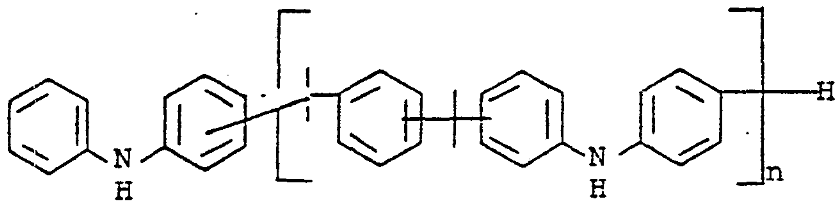

507 g (3 Mol) Diphenylamin und 194 g (1 Mol) α,α'-Dihydroxy-m/p-diisopropylbenzol (Molverhältnis 3/2) werden aufgeschmolzen unter $N_2$ vereinigt, bei 60-70° mit 20 g säureaktivierter Tonerde versetzt und langsam unter Rühren weiter erhitzt, bis ab 110° $H_2O$ abgespalten wird. Nachdem alles Wasser (insgesamt 36 g) übergegangen ist, erhitzt man noch für 2 h von 160 auf 180°, filtriert durch eine beheizte Drucknutsche und destilliert bei 8-10 mbar bis zu einer Sumpftemperatur von 240° und einer Kopftemperatur von 160° Monomere ab. Man erhält 451 g eines klaren spröden schwach hellbraunen Harzes.

**Beispiel 31**

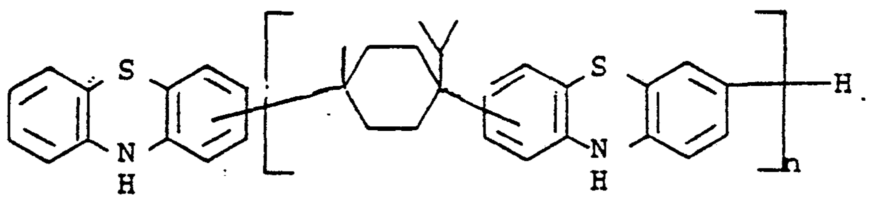

Zu einer Schmelze von 199 g (1 Mol) Phenothiazin gibt man unter $N_2$ bei 180-185° 10 g säureaktivierter Bleicherde und tropft in 1 h unter Rühren 136 g (1 Mol) Limonen hinzu. Nach weiteren 2 h Rühren bei 180° nimmt man das Gemisch in Xylol auf, filtriert durch eine Drucknutsche und dampft das klare gelbe Filtrat bei Wasserstrahlvakuum bis zu einer Sumpftemperatur von 190° bei 25 mbar ein. Man erhält ein gelbbraunes sprödes Harz (310 g). Laut Gelchromatogramm betragen die Molekulargewichte bis etwa 2800.

**Beispiel 32**

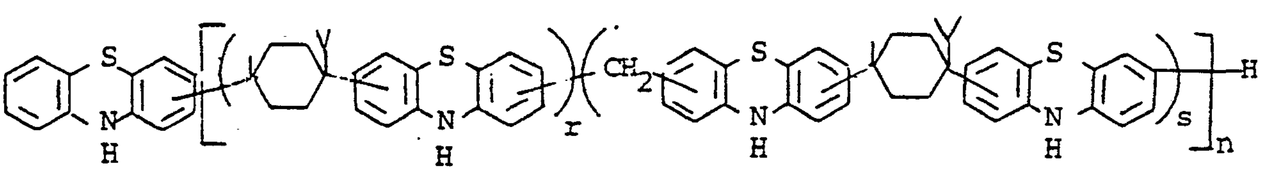

100 g des Produktes aus Beispiel 31 in 100 g o-Dichlorbenzol werden unter Rühren und Stickstoff mit 3 g säureaktivierter Tonerde und 9 g 35 %igem Formalin versetzt und 2 h unter Rückfluß gekocht. Unter Abdestillieren von Wasser wird die Sumpftemperatur auf 180° gebracht, 2 h bei dieser Temperatur gehalten, das Gemisch filtriert und bis 200°/18 mbar eingedampft. Es bleiben 101 g eines hellbraunen spröden Harzes. (Molekulargewicht bis 3500 - gelchromatographisch -).

**Beispiel 33**

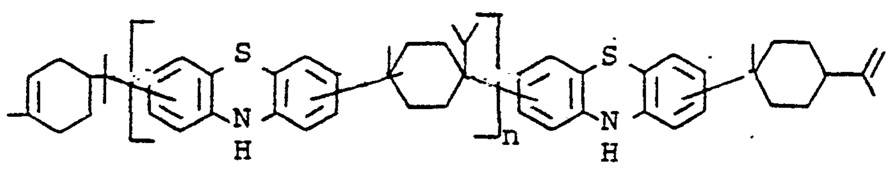

Zu einem Gemisch von 199 g (1 Mol) Phenothiazin, 200 g o-Dichlorbenzol und 10 g säureaktivierter Tonerde tropft man unter Stickstoff und Rühren 150 g (1,1 Mol) Limonen in 2 h bei 170-180° zu, läßt 3 h nachreagieren, filtriert und dampft bis 200°/9 mbar im Sumpf ein. Es werden 340 g eines gelbbraunen spröden Harzes erhalten (mit Molekulargewichten bis zu 3400).

**Anwendungsbeispiele für die Stabilisatoren bei der Schaumstoffherstellung**

Versuchsdurchführung (allgemeines Verfahren)

Für die praktische Erprobung der erfindungsgemäßen Stabilisatoren wurden jeweils Schaumstoffe mit den Abmessungen L = 1,8 m; B = 1,0 m; H = ca. 1,2 m hergestellt.

Die gegebenenfalls pulverisierten Stabilisatoren wurden in den jeweiligen Polyetherpolyolen zu 1 Gew.-% eingewogen und unter fortlaufendem Rühren so lange erhitzt (maximal auf 80°C), bis sich die Stabilisatoren vollkommen gelöst hatten. Nach dem Abkühlen wurde dieser Stabilisator/Polyether-Stammansatz dann dem eigentlichen Polyetherpolyolreaktionsansatz in derjenigen Menge zugegeben, die für eine Konzentration von 0,1 bzw. 0,2 Gew.-% Stabilisator notwendig war. Die weiteren Zusatzkomponenten (Schaumstabilisatoren und Aminkatalysatoren) wurden in einem Polyetherpolyol-Stammansatz eingewogen und bei ca. 1800 U/Min. verrührt. Dieses Stammansatzgemisch wurde dann dem Polyetherpolyol gleichfalls vor Ort (kurz vor der Polyisocyanatzugabe) zugemischt und 60 Sekunden bei 1800 U/Min. homogenisiert. Anschließend wurden bei gleicher Drehzahl gegebenenfalls Zinnkatalysator und das Wasser 60 Sekunden lang eingerührt.

Für die Polyisocyanatzugabe wurde der Behälter mit dem Polyolgemisch in eine mit Folie ausgekleidete Kiste der obengenannten Ausmaße L = 1,8 m, B = 1,0 m, H = ca. 1,2 m gebracht. Die erforderliche Polyisocyanatmenge wurde mit dem alle Zusätze enthaltenden Polyetherpolyol mittels eines speziellen Luftrührers bei ca. 1000 U/Min. 10 Sekunden lang vermischt und dann das Reaktionsgemisch kurz über dem Boden der mit Folie ausgekleideten Kiste ausgegossen. Die Mischung schäumt unter Verfestigung zum Schaumstoff auf.

Nach ca. 24 Stunden wurden die Schaumstoffblöcke zur Beurteilung der Kernverfärbung aufgeschnitten und die Kernverfärbung/Kernverbrennung beurteilt.

Die erfindungsgemäßen Stabilisatoren wurden in bis zu 3 verschiedenen Schaumstoffrezepturen eingesetzt. Die Rezepturen unterschieden sich einerseits im Wassergehalt (6,5 und 8,0 Gew.-%) und andererseits in der Art der Katalyse.

**Rezeptur A**

100 Gew.-Teile eines trifunktionellen, auf Trimethylolpropan gestarteten polypropylenetherpolyols der OH-Zahl 56, mit 0,20 Gew.-% Ionol vorstabilisiert

1,5 Gew.-Teile eines handelsüblichen Polysiloxan-Polyalkylenglykol-Blockcopolymers als Weichschaumstabilisator

0,15 Gew.-Teile Zinn(II)-octoat

0,1 Gew.-Teile eines handelsüblichen Aminkatalysatorgemischs (®Desmorapid PS 207, Handelsprodukt der Bayer AG)

6,5 Gew.-Teile Wasser

75,1 Gew.-Teile Toluylendiisocyanat (Gemisch aus 2,4-und 2,6-Diisocyanato-toluol im Verhältnis 80:20)

NCO-Index = 105

0,1 Gew.-Teile eines zur Prüfung stehenden Stabilisators.

**Rezeptur B**

100 Gew.-Teile eines trifunktionellen Polyetherpolyols wie in Rezeptur A
1,5 Gew.-Teile eines handelsüblichen Polysiloxan-Polyalkylenglykol-Blockcopolymers als Weichschaumstabilisator
0,15 Gew.-Teile Zinn(II)-octoat
0,15 Gew.-Teile eines handelsüblichen Aminaktivators (33 %ige Lösung von Triethylendiamin in Dipropylenglykol)
0,3 Gew.-Teile Dimethylethanolamin
6,5 Gew.-Teile Wasser
78,7 Gew.-Teile Toluylendiisocyanat (Gemisch aus 2,4-und 2,6-Diisocyanatotoluol im Verhältnis 80:20)
NCO-Index = 110
0,1 Gew.-Teile eines zur Prüfung stehenden Stabilisators.

**Rezeptur C**

100 Gew.-Teile eines trifunktionellen Polyetherpolyols wie in Rezeptur A
1,8 Gew.-Teile eines handelsüblichen Polysiloxan-Polyalkylenglykol-Blockcopolymers als Weichschaumstabilisator
0,17 Gew.-Teile Zinn(II)-octoat
0,2 Gew.-Teile eines handelsüblichen Aminkatalysatorgemischs (® Desmorapid PS 207, Handelsprodukt der Bayer AG)
8,0 Gew.-Teile Wasser
94,6 Gew.-Teile Toluylendiisocyanat (Gemisch aus 2,4-und 2,6-Diisocyanatotoluol im Verhältnis 80:20)
NCO-Index = 110
\+ 0,1 Gew.-Teile eines zur Prüfung stehenden Stabilisators.

Tabelle 1

| Anwendungs-beispiel Nr. | Prod.-Gruppe (allg. Formel) | Hers.-Beisp. Nr. | Rezeptur | PU-Schaumstoff-Beurteilung hinsichtlich Kernverfärbung: + 0,1 Gew.-% Dioctyl-diphenylamin (Vergleich nach Stand der Technik) | | + 0,1 Gew.-% erfindungsgemäßer Stabilisator (erfindungsgemäß) | |
|---|---|---|---|---|---|---|---|
| | | | | visulle Beurteilung | Braunskala | visuelle Beurteilung | Braunskala |
| 35 | I | 3 | A | sehr starke Kernverfärbung | 7 | schwache Kernverfärbung | 2 |
| 36 | | 3 | B | sehr starke Kernverfärbung | 7 | Kernverfärbung | 4 |
| 37 | | 3 | C | Selbstentzündung (SE) | SE | Kernverfärbung | 5 |
| 38 | II | 29 | A | sehr starke Kernverfärbung | 7 | Kernverfärbung | 4 |
| 39 | | 29 | B | sehr starke Kernverfärbung | 7 | Kernverfärbung | 5 |
| 40 | II | 5 | A | sehr starke Kernverfärbung | 7 | Kernverfärbung | 4 |
| 41 | | 5 | B | sehr starke Kernverfärbung | 7 | Kernverfärbung | 5 |

Tabelle 1 (Fortsetzung)

| Anwendungs-beispiel Nr. | Prod.-Gruppe (allg. Formel) | Hers.-Beisp. Nr. | Rezep-tur | PU-Schaumstoff-Beurteilung hinsichtlich Kernverfärbung: + 0,1 Gew.-% Dioctyl-diphenylamin (Vergleich nach Stand der Technik) | | + 0,1 Gew.-% erfindungsgemäßer Stabilisator (erfindungsgemäß) | |
|---|---|---|---|---|---|---|---|
| | | | | visulle Beurteilung | Braunskala | visuelle Beurteilung | Braunskala |
| 42 | III | 1 | A | sehr starke Kernverfärbung | 7 | schwache Kernverfärbung | 2 |
| 43 | | 1 | B | sehr starke Kernverfärbung | 7 | schwache Kernverfärbung | 3 |
| 44 | | 1 | C | Selbstentzündung | SE | Kernverfärbung | 5 |
| 45 | III | 2 | A | sehr starke Kernverfärbung | 7 | schwache Kernverfärbung | 2 |
| 46 | | 2 | B | sehr starke Kernverfärbung | 7 | schwache Kernverfärbung | 3 |
| 47 | | 2 | C | Selbstentzündung | SE | starke Kernverfärbung | 6 |
| 48 | III | 10 | A | sehr starke Kernverfärbung | 7 | schwache Kernverfärbung | 2 |
| 49 | | 10 | B | sehr starke Kernverfärbung | 7 | schwache Kernverfärbung | 3 |
| 50 | | 10 | C | Selbstentzündung | SE | Kernverfärbung | 5 |

Tabelle 1 (Fortsetzung)

| Anwendungs-beispiel Nr. | Prod.-Gruppe (allg. Formel) | Hers.-Beisp. Nr. | Rezep-tur | PU-Schaumstoff-Beurteilung hinsichtlich Kernverfärbung: + 0,1 Gew.-% Dioctyl-diphenylamin (Vergleich nach Stand der Technik) | | + 0,1 Gew.-% erfindungsgemäßer Stabilisator (erfindungsgemäß) | |
|---|---|---|---|---|---|---|---|
| | | | | visulle Beurteilung | Braunskala | visuelle Beurteilung | Braunskala |
| 51 | IV | 14 | A | sehr starke Kernverfärbung | 7 | keine Kernverfärbung | 0 |
| 52 | | 14 | B | sehr starke Kernverfärbung | 7 | keine Kernverfärbung | 0 |
| 53 | | 14 | C | Selbstentzündung | SE | keine Kernverfärbung | 0 |
| 54 | IV | 8 | A | sehr starke Kernverfärbung | 7 | keine Kernverfärbung | 0 |
| 55 | | 8 | B | sehr starke Kernverfärbung | 7 | keine Kernverfärbung | 0 |
| 56 | | 8 | C | Selbstentzündung | SE | keine Kernverfärbung | 0-1 |
| 57 | IV | 6 | A | sehr starke Kernverfärbung | 7 | keine Kernverfärbung | 0 |
| 58 | | 6 | B | sehr starke Kernverfärbung | 7 | keine Kernverfärbung | 0-1 |
| 59 | | 6 | C | Selbstentzündung | SE | keine Kernverfärbung | 0-1 |

Tabelle 1 (Fortsetzung)

| Anwendungs-beispiel Nr. | Prod.-Gruppe (allg. Formel) | Hers.-Beisp. Nr. | Rezep-tur | PU-Schaumstoff-Beurteilung hinsichtlich Kernverfärbung: + 0,1 Gew.-% Dioctyl-diphenylamin (Vergleich nach Stand der Technik) | | + 0,1 Gew.-% erfindungsgemäßer Stabilisator (erfindungsgemäß) | |
|---|---|---|---|---|---|---|---|
| | | | | visulle Beurteilung | Braunskala | visuelle Beurteilung | Braunskala |
| 60 | IV | 31 | A | sehr starke Kernverfärbung | 7 | keine Kernverfärbung | 0 |
| 61 | | 31 | B | sehr starke Kernverfärbung | 7 | keine Kernverfärbung | 0 |
| 62 | | 31 | C | Selbstentzündung | SE | keine Kernverfärbung | 0 |
| 63 | V | 28 | A | sehr starke Kernverfärbung | 7 | keine Kernverfärbung | 0 |
| 64 | | 28 | B | sehr starke Kernverfärbung | 7 | keine Kernverfärbung | 0 |
| 65 | | 28 | C | Selbstentzündung | SE | schwache Kernverfärbung | 2 |

Bemerkung:

Nach der Braunskala bedeutet 0 = keine Kernverfärbung

1-7 = zunehmende Kernverfärbung von sehr schwach bis sehr stark

SE = Selbstentzündung

**Ergebnisse:**

Die Ergebnisse der einzelnen Stabilisierungsversuche (siehe Anwendungsbeispiele 35 bis 65 in Tabelle 1) zeigen die sehr starke Wirksamkeit der erfindungsge-mäßen Stabilisatoren.

Mit einem gemäß dem Stand der Technik optimalen und weiterhin geübten Oxidationsschutz erhält man in Rezepturen mit relativ hohem Wasseranteil mit Sicherheit starke Kernverfärbung und bei besonders kritischen Ansätzen (Rezeptur C) unvermeidbar Selbstentzündung der Schaumstoffe. Dagegen zeigen die erfindungsgemäßen Stabilisatoren wesentlich günstigeres Verhalten. Mittels der Produktgruppen der allgemeinen Formel I, III, IV und V lassen sich Stabilisierungen erzielen, welche die Selbstentzündung der Schaumstoffe mit Sicherheit ausschließen. Besonders günstige Werte zeigen innerhalb der Werte der bevorzugten Produktgruppen III, IV und V die Stabilisatoren der Gruppe IV. Mittels dieser Stabilisatoren läßt sich nicht nur eine Selbstentzündung sicher ausschließen, sondern es tritt auch keine Kernverfärbung der Schaumstoffe ein.

**Patentansprüche**

1. Stabilisatorhaltige Reaktivkomponenten zur Herstellung von nicht oder wenig kernverfärbenden Polyurethanschaumstoffen auf der Basis von Polyisocyanaten, Polyolen, sowie gegebenenfalls Wasser, Treibmitteln, Katalysatoren, weiteren Stabilisatoren und üblichen Zuschlagstoffen, dadurch gekennzeichnet, daß sie als Stabilisatoren monomere und/oder oligomere Derivate der Diphenylaminreihe einschließlich der Phenothiazinreihe in stabilisierenden Mengen von 0,02 bis 5 Gew.-% Verbindungen

der allgemeinen Struktur 1

I

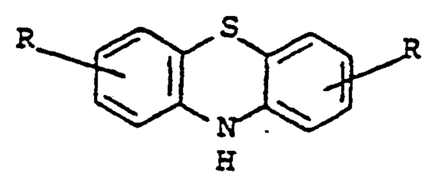

in der

R = $C_7$-$C_{18}$-Aralkyl bedeutet (die Zahl der C-Atome gibt die Gesamtzahl im Arylrest inclusive seiner Alkylsubstituenten an);

der allgemeinen Struktur II

II

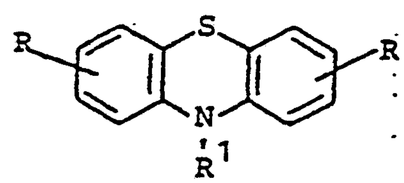

in der

R die obengenannte Bedeutung hat,

$R^1$ = $C_1$-$C_{18}$-Alkyl, $C_5$-$C_{12}$-Cycloalkyl und -Cycloalkenyl, $C_7$-$C_{18}$-Aralkyl, die gegebenenfalls durch OH-, SH-, Ether-, Thioether-, Carbonester-, Carbonamid- und Carboxyl-Gruppen substituiert oder durch solche Gruppen (außer OH, SH und COOH) und olefinische Doppelbindungen unterbrochen sein können,

ferner ein Rest

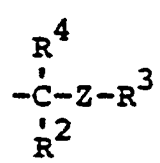

ist, worin

$R^2$, $R^3$ und $R^4$ gleich oder verschieden sind und H, $C_1$-$C_{12}$-Alkyl, $C_5$-$C_{12}$-Cycloalkyl und -Cycloalkenyl, $C_7$-$C_{12}$-

Aralkyl,
$R^2$ außerdem gegebenenfalls substituiertes Aryl ist und mit $R^4$ und dem zentralen C-Atom zusammen einen 5- bis 12-gliedrigen aliphatischen Ring bilden kann,
Z O, S, NH, $NR^5$, worin $R^5 = R^2$ ist, oder ein Rest der Formel CO-A-$R^2$, in dem A eine einfache Bindung, S, O, NH oder $NR^2$ bedeutet, wobei hier und folgend für $R^2$ die Ringbildung mit $R^4$ entfällt,
Z mit $R^3$ zusammen den Rest

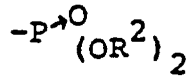

und
$R^1$ neben der genannten Bedeutung ferner den Rest

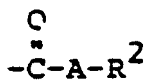

darstellt;
der allgemeinen Struktur III

III 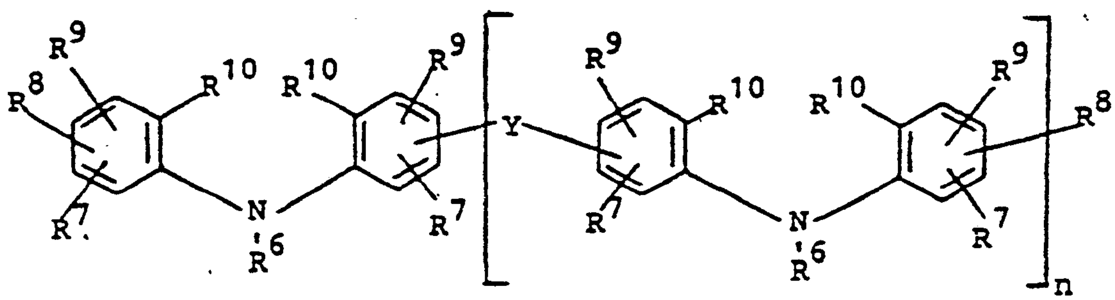

in der
$R^6$ = H oder $R^1$,
$R^7$, $R^9$ und $R^{10}$ gleich oder verschieden sind und H, $CH_3$ oder $C_2H_5$ bedeuten,
$R^8$ = H, Benzyl-, Styryl-, α-Methylstyryl-, tert.-Butyl-, tert.-Amyl-, Isononyl-, Cyclohexyl-, Methylcyclohexyl-,

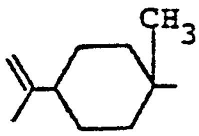 , 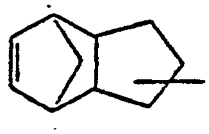 , 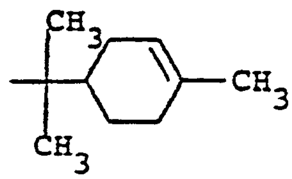 , 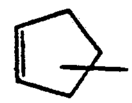

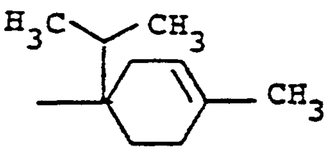 , 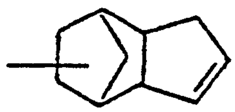 , 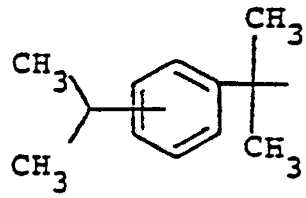 ,

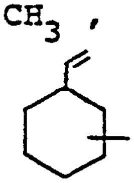 , 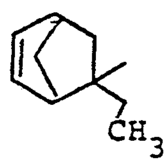

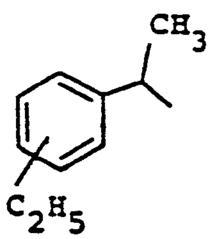 , 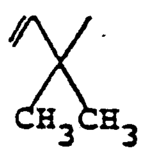 , 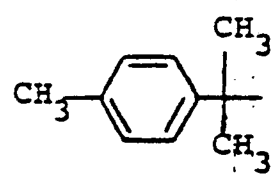 ,

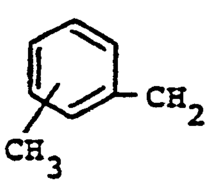 und 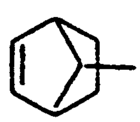 bedeuten,

Y = 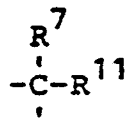

ist, wobei $R^7$ die obengenannten Bedeutung hat und wobei $R^{11}$ $C_1$-$C_7$-Alkyl, Cyclohexyl, Cyclohexenyl, Aryl ist, weiter

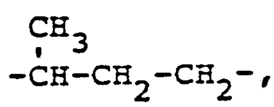 , 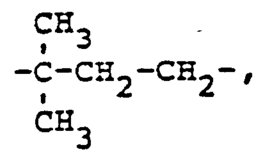 , 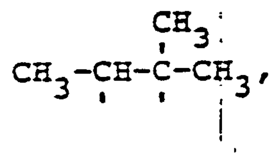 ,

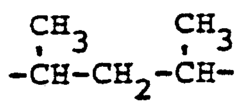 , 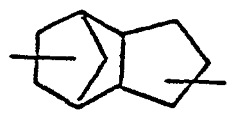 , 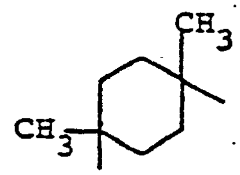 , 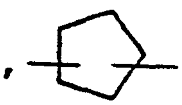 ,

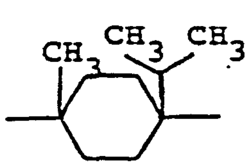 , 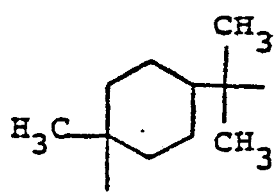 , 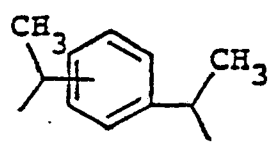 , 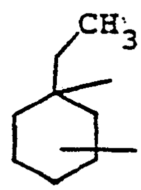 ,

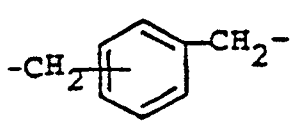 , 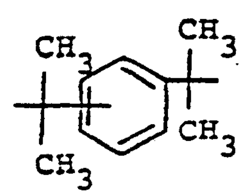 , 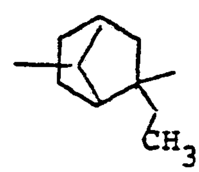 ist,

ist,
und bis zu 60 Mol-% auch -S-, $-CH_2-$, $-CH_2-S-CH_2$ oder $-CH_2-0-CH_2-$ sein kann, und
n eine ganze Zahl von 1 bis 29 darstellt, jedoch ausgeschlossan Produkte entsprechend der Umsetzung von Diphenylamin und Aceton($R^6$ bis $R^{10}$ = H;

Y = 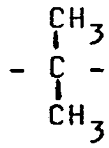 ;

der allgemeinen Struktur IV

IV 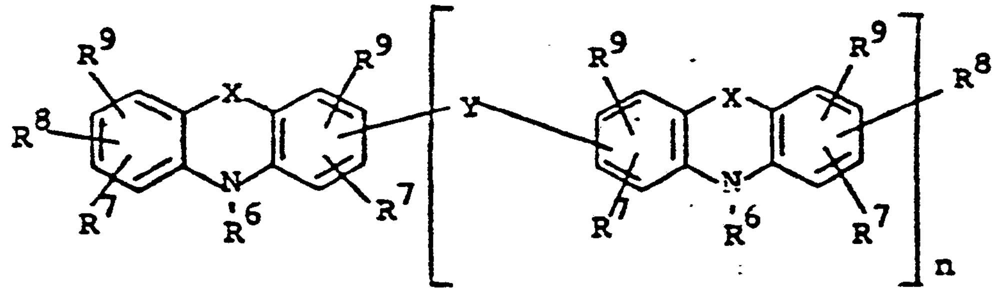

in der
X -S- bedeutet;
und der allgemeinen Struktur V

V

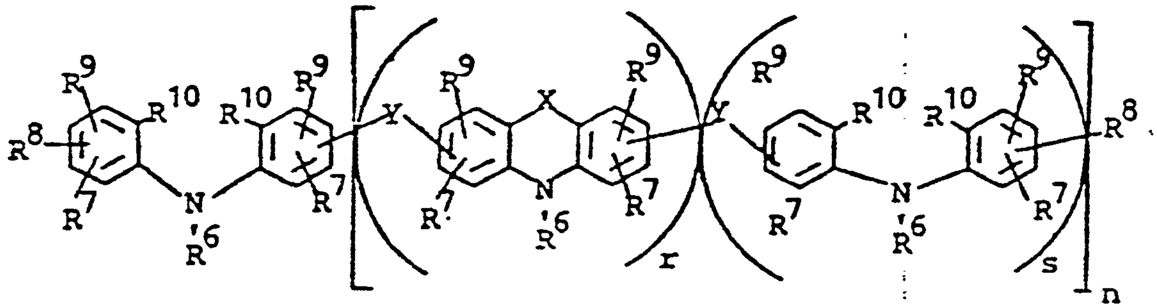

in der
r und s ganze Zahlen von 1 bis 29 darstellen,
enthalten.

2. Verbindungen der allgemeinen Struktur III

III 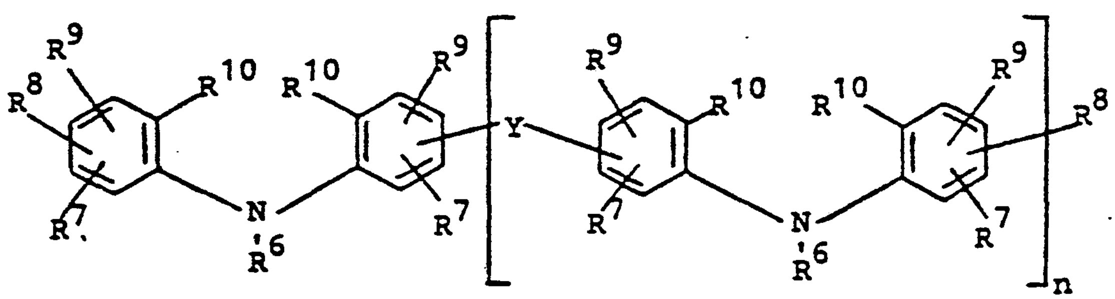

in der
$R^6$ = H oder $R^1$,
$R^7$, $R^9$ und $R^{10}$ gleich oder verschieden sind und H, $CH_3$ oder $C_2H_5$ bedeuten,
$R^8$ = H, Benzyl-, Styryl-, α-Methylstyryl-, tert.-Butyl-, tert.-Amyl-, Isononyl-, Cyclohexyl-, Methylcyclohexyl-,

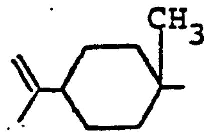 , 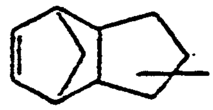 , 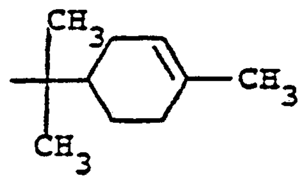 ,

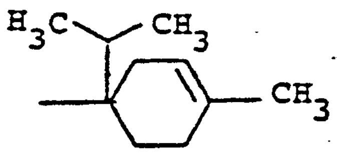 , 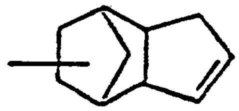 , 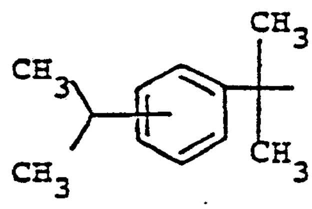 ,

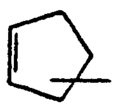 , 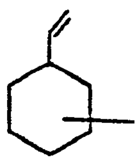 , 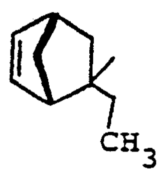

; 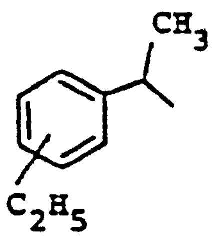 , 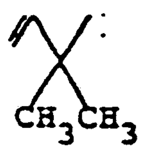 , 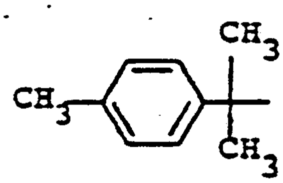 ,

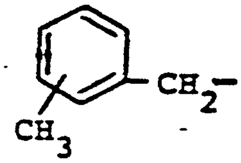 und 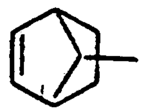 bedeuten,

Y = 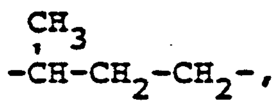, 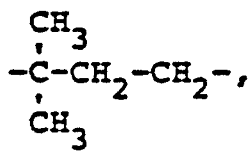, 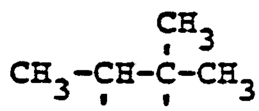,

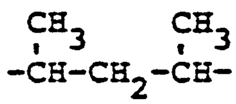, 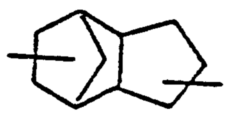, 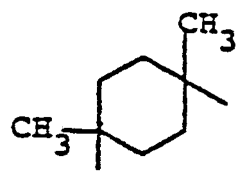, 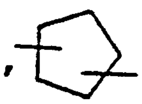,

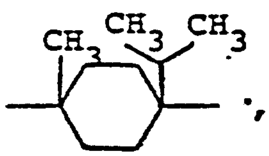, 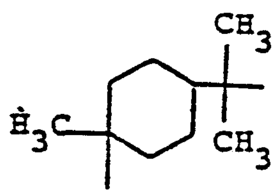, 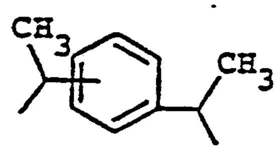, 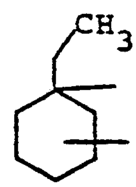,

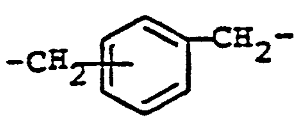, 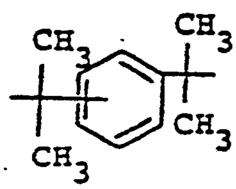, 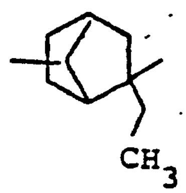 ist,

und bis zu 60 Mol-% auch -S-, $-CH_2-$, $-CH_2-S-CH_2$ oder $-CH_2-0-CH_2-$ sein kann, und n eine ganze Zahl von 1 - 29 darstellt;
jedoch ausgeschlossen Verbindungen, bei denen $R^6$ bis $R^{10}$ = H und

Y = 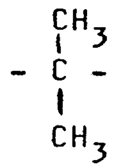

ist;
der allgemeinen Struktur IV

IV 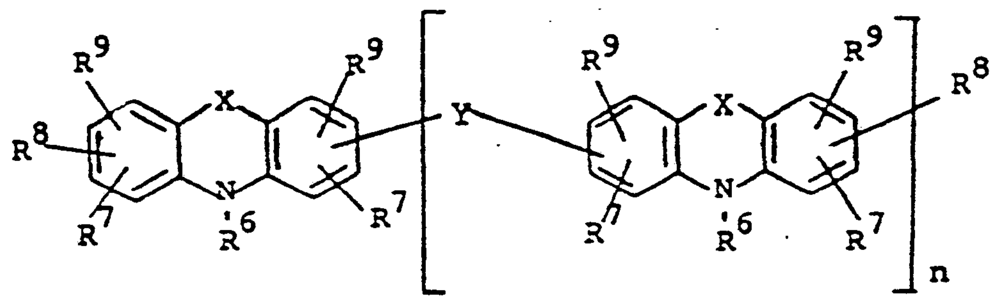

in der

Y = 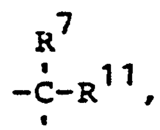

wobei $R^7$ die obengenannte Bedeutung hat, vorzugsweise aber H ist und wobei $R^{11}$ $C_1$-$C_7$-(vorzugsweise $C_1$-$C_4$-) Alkyl, Cyclohexyl, Cyclohexenyl und Aryl ist, ferner die unter III angegebene Bedeutung hat,
X = - S
bedeutet;
und der allgemeinen Struktur V

V

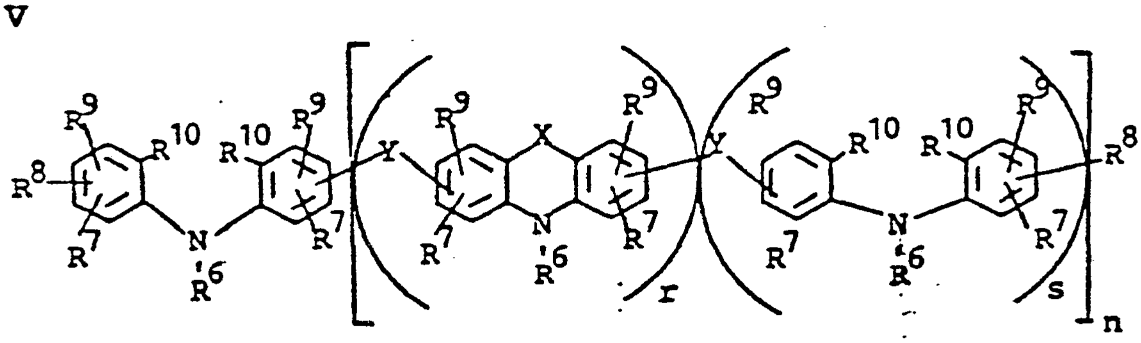

in der
r und s ganze Zahlen von 1 - 29 darstellen.

3. Verfahren zur Herstellung von monomeren und/oder oligomeren Gemischen der Verbindungen der Strukturen III bis V nach Anspruch 2, dadurch gekennzeichnet, daß man aromatische Amine der Formeln

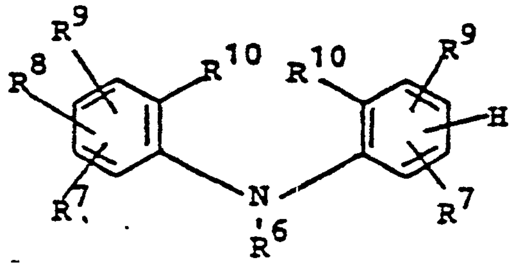

und

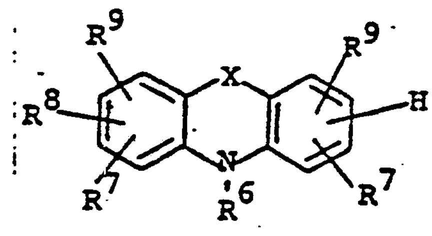

wobei die Reste $R^6$, $R^7$, $R^8$, $R^9$, $R^{10}$ und X die in Anspruch 2 angeführte Bedeutung haben, mit bifunktionellen Verbindungen der Formeln

Hal-Y-Hal (Hal = Halogen)
HO-Y-OH
$R^7O$-Y-$OR^7$
$R^7COO$-Y-$OCOR^7$,

wobei der Rest Y die in Anspruch 2 angeführte Bedeutung hat, bzw. aus diesen durch Abspaltung des Restes HOH, $HOR^7$, $HOCOR^7$ oder H-Hal sich bildenden Bisolefinen,

in Gegenwart von starken Säuren mit einem $pk_s$-Wert von kleiner als 2 bei Temperaturen zwischen 50-300°C umsetzt und die Reste $R^8$ und $R^6$ (je ungleich H) vor, während oder nach der obigen Umsetzung der Amine einführt.

**Claims**

1. Stabiliser-containing reactive components for the production of polyurethane foams with little or no tendency towards core discolouration based on polyisocyanates, polyols and, optionally, water, blowing agents, catalysts, other stabilisers and standard additives, characterised in that they contain as stabilisers monomeric and/or oligomeric derivacivesof the diphenylamine series, including the phenothiazine series, in stabilising quantiries of from 0.02 to 5% by weight of compounds

of the general formula 1

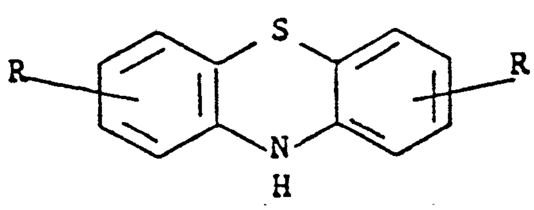

I

in which

R denotes $G_7$-$C_{18}$-aralkyl (the number of carbon atoms indicates the total number in the aryl radical, including its alkyl substituents);

of the general formula II

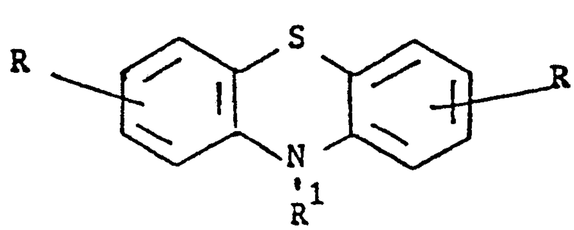

in which

R has the abovementioned meaning,

$R^1$ is $C_1$-$C_{18}$-alkyl, $C_5$-$C_{12}$-cycloalkyl and -cycloalkenyl, $C_7$-$C_{18}$-aralkyl which may optionally be substituted by

OH-, SH-, ether, thioether, carboxylic ester, carboxamide and carboxyl groups or may be incerrupted by such groups (apart from OH SH and COOH) and olefinic double bonds, and is also a radical

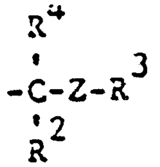

wherein

$R^2$, $R^3$ and $R^4$ are the same or different and

H, $C_1$-$C_{12}$-alkyl, $C_5$-$C_{12}$-cycloalkyl and-cycloalkenyl and $C_7$-$C_{12}$-aralkyl,

in addition to which $R^2$ is optionally substituted aryl and, together with $R^4$ and the central C-atom, may form a 5- to 12-membered aliphatic ring.

Z is O, S, NH or $NR^5$ wherein $R^5 = R^2$,

or a radical of the formula CO-A-$R^2$, in which A is a single bond, S, O, NH or $NR^2$; both here and in the following,

$R^2$ does not form a ring with $R^4$,

Z together with $R^3$ represents the radical

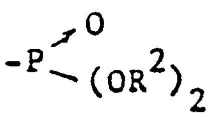

and

$R^1$ in addition to the meaning given above also represents the radical

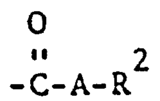 ;

of the general formula III

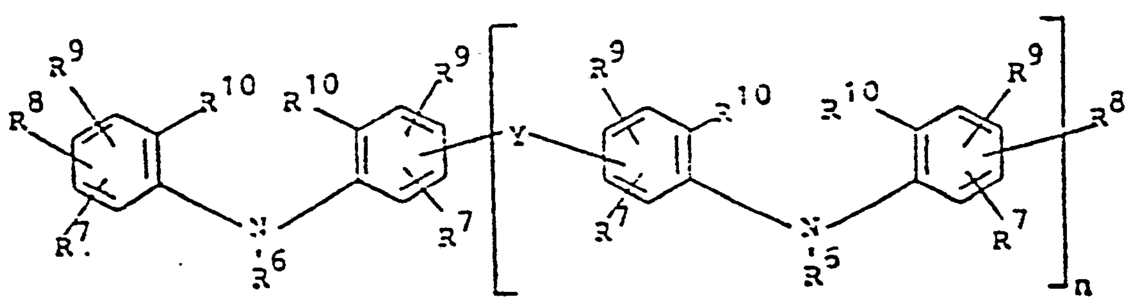

in which

$R^6$ is H or $R^1$,

$R^7$, $R^9$ and $R^{10}$ are the same or different and denote H, $CH_3$ or $C_2H_5$,

$R^8$ = H, benzyl, styryl, α-methyl styryl, tert.-butyl tert.-amyl, isononyl cyclohexyl methyl cyclohexyl,

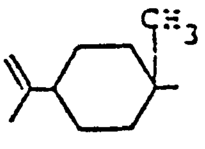 , 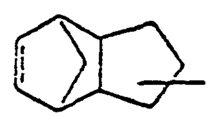 , 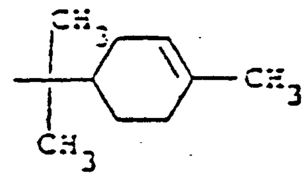 , 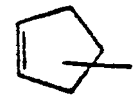

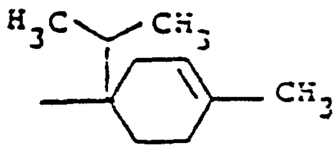 , 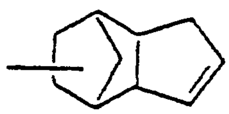 , 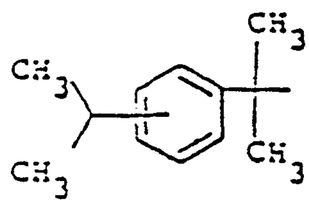 ,

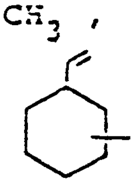 , 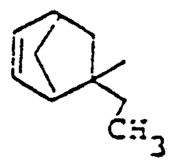

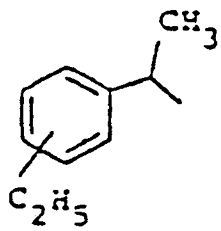 , 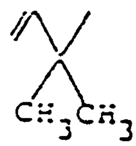 , 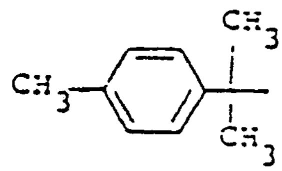 ,

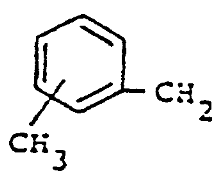 and 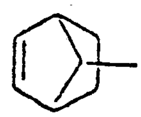 ,

Y is

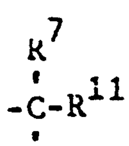

wherein $R^7$ has the atovementioned meaning and wherein $R^{11}$ is $C_1$-$C_7$-alkyl, cyclohexyl, cyclohexenyl, aryl, and is also

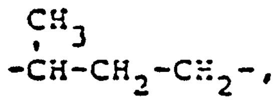, 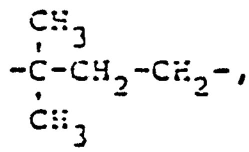, 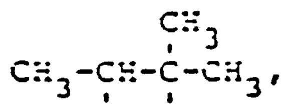,

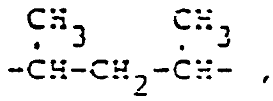, 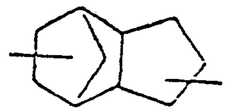, 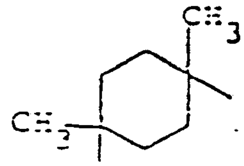, 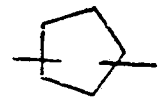,

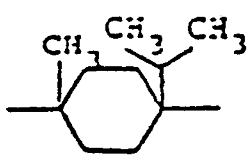, 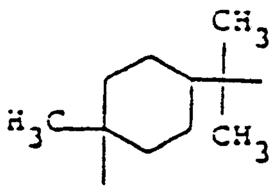, 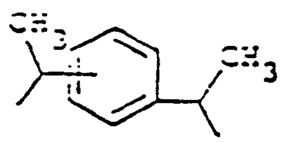, 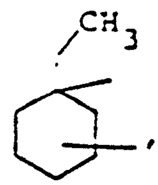,

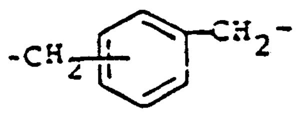, 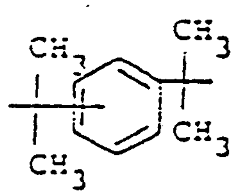, 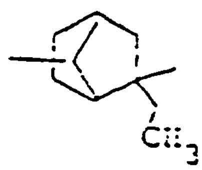

and up to 60 mole percent may also be -S-, $-CH_2-$, $-CH_2-S-CH_2-$or $-CH_2-O-CH_2-$ and n is an integer of from 1 to 29, but excluding products conforming to the reaction of diphenylamine and acetone ($R^6$ to $R^{10}$ = H;

Y = 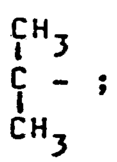 ;

of the general formula IV

IV 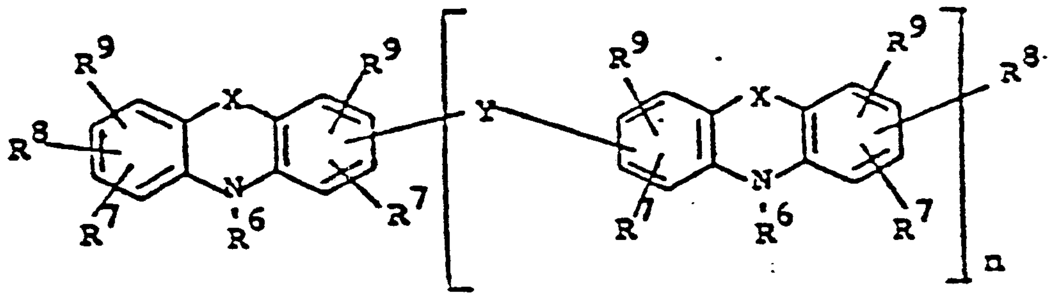

in which
X denotes - S -;
and of the general formula V

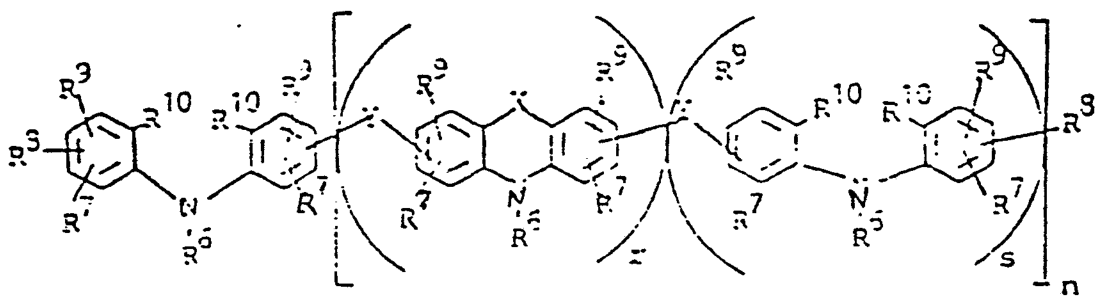 V

in which
r and s represent integers of from 1 to 29.
2. Compounds of the general formula III

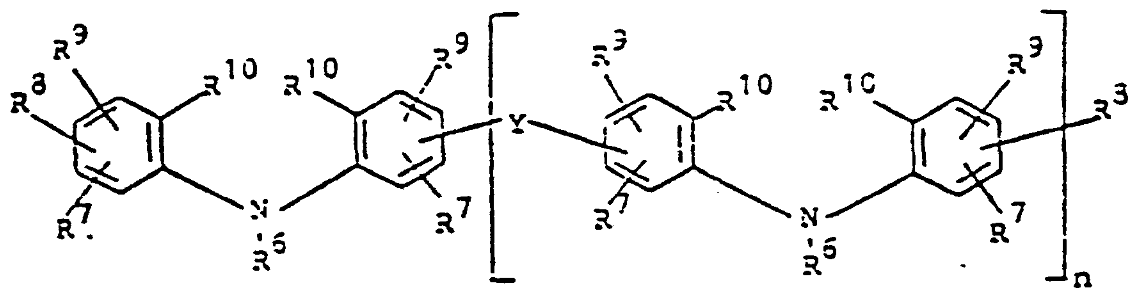

in which
$R^6$ is H or $R^1$,
$R^7$ $R^9$ and $R^{10}$ are the same or different and denote H, $CH_3$ or $C_2H_5$,
$R^8$ denotes H, benzyl, styryl, α-methyl styryl, tert.-butyl, tert.-amyl, isononyl, cyclohexyl, methyl cyclohexyl,

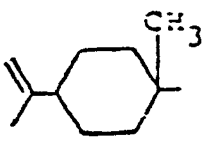 , 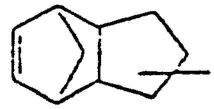 , 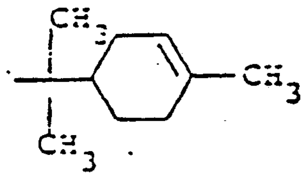 ,

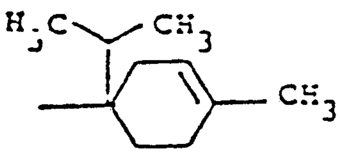 , 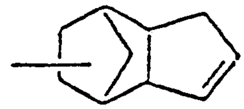 , 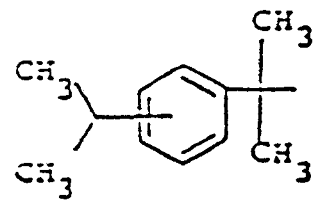 ,

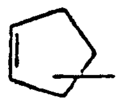 , 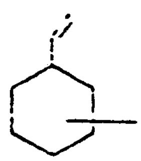 , 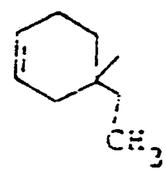

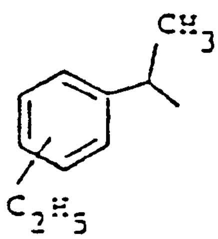 , 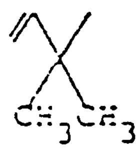 , 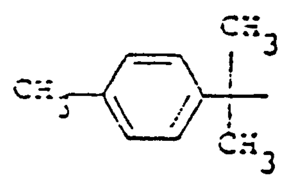 ,

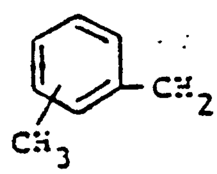 and 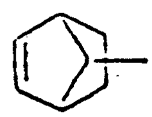 ,

Y is

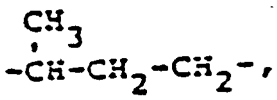, 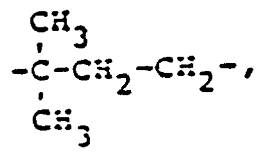, 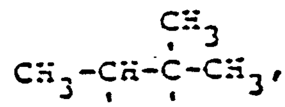,

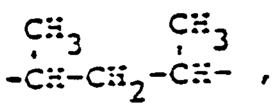, 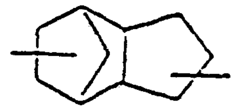, 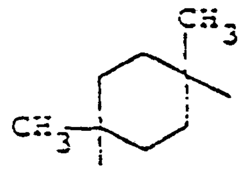, 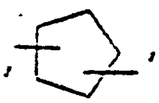,

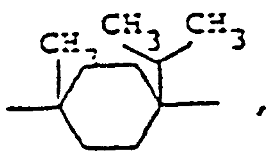, 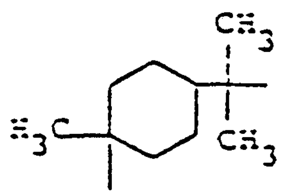, 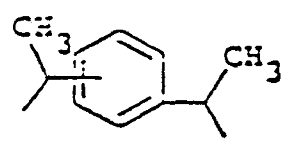, 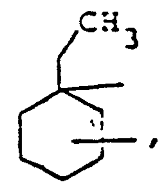,

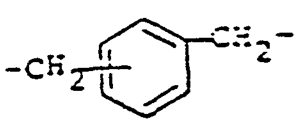, 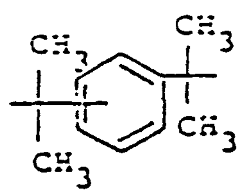, 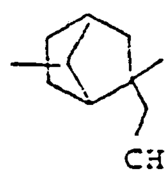

and, up to 60 mole percent, may also be -S-, $-CH_2-$, $-CH^2-S-CH^2-$or $-CH_2-O-CH_2-$, and n represents an integer of from 1 - 29; but excluding compounds in which $R^6$ to $R^{10}$ = H and

Y = 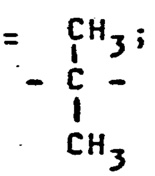

of the general formula IV

IV 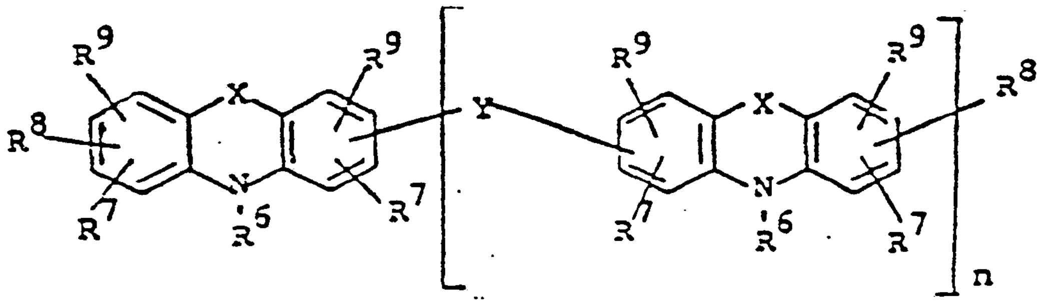

in which
Y denotes

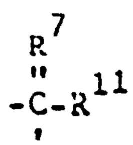

wherein $R^7$ has the abovementioned meaning, but is preferably H, and wherein $R^{11}$ is $C_1$-$C_7$-(preferably $C_1$-$C_4$-) alkyl, cyclohexyl, cyclohexenyl and aryl, and in addition has the meaning given under III,
X denotes - S -;
and of the general formula V

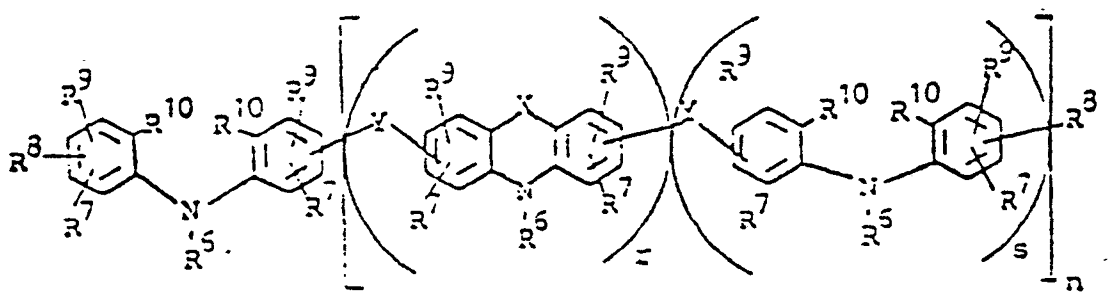 V

in which
r and s represent integers of from 1 to 29.

3. A prcess for the production of monomeric and/or oligomeric mixtures of the compounds of formulae III to V according to Claim 2, characterised in that aromatic amines of the formulae

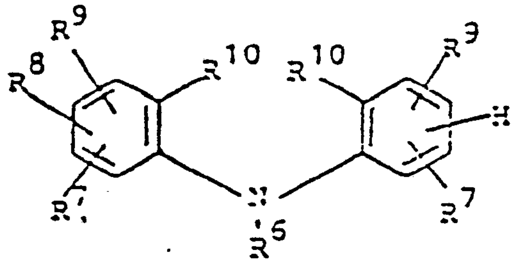 and 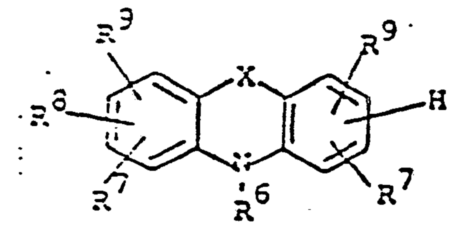

wherein the radicals $R^6$, $R^7$, $R^8$, $R^9$, $R^{10}$ and X have the meaning given in Claim 2,
are reacted with bifunctional compounds of the formulae
Hal-Y-Hal (Hal = halogen)

HO-Y-OH
$R^7O$-Y-$OR^7$
$R^7COO$-Y-$OCOR^7$
in which the radical Y has the meaning given in Claim 2, or with bis-olefins formed from these compounds by elimination of the radical HOH, $HOR^7$ $HOCOR^7$ or H-Hal, at temperatures between 50 - 300°C in the presence of strong acids having a pk -value of less than 2 and the radicals $R^8$ and $R^6$ (of which neither reoresents H) are introduced before, during or after the above reaction of the amines.

**Revendications**

1. Composants réactifs contenant un stabilisant pour la production de mousses de polyuréthanne à changement de couleur nul ou faible à coeur, à base de polyisocyanates, de polyols ainsi que, le cas échéant, d'eau, d'agents porogenes, de catalyseurs, d' autres stabilisants et d'additifs classiques, caractérisés en ce qu'ils contiennent comme stabilisants des dérivés monomériques et/ou oligomériques de la série de la diphénylamine y compris la série de la phénothiazine,en quantités stabilisantes de 0,02 à 5 % en poids de composés
de formule générale I

I .

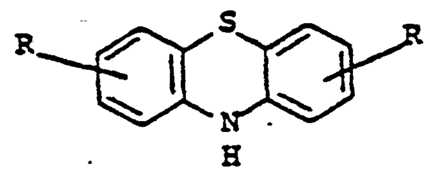

dans laquelle
R est un reste aralkyle en $C_7$ à $C_{18}$ (le nombre d'atomes de carbone indique le nombre total dans le reste aryle, y compris ses substituants alkyle)
de formule générale II

II

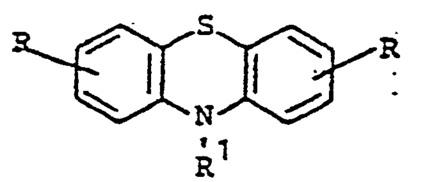

dans laquelle
R a la définition donnée ci-dessus,
$R^1$ est un groupe alkyle en $C_1$ à $C_{18}$, cycloalkyle et cycloalcényle en $C_5$ à $C_{12}$, aralkyle en $C_7$ à $C_{18}$, qui peuvent etre substitués, le cas échéant,par des groupes OH, SH, éther, thioéther, ester carboxylique, carboxamide et carboxyle ou interrompus par de tels groupes (hormis OH, SH et COOH) et des doubles liaisons oléfiniques,
en outre un reste de formule

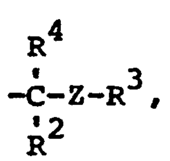

dans laquelle
$R^2$, $R^3$ et $R^4$ sont identiques ou différents et représentent H, un groupe alkyle en $C_1$-à $C_{12}$, un groupe cycloalkyle et cycloalcényle en $C_5$ à $C_{12}$, aralkyle en $C_7$ à $C_{12}$, $R^2$ est en outre un groupe aryle éventuellement substitué et peut former, conjointement avec $R^4$ et l'atome central de carbone, un noyau aliphatique de 5 à 12 chaînons,
Z représente O, S, NH, $NR^5$, $R^5$ représentant $R^2$, ou un reste de formule CO-A-$R^2$ dans lequel A représente une

liaison simple, S, O, NH ou $NR^2$, auquel cas et par la suite la formation d'un noyau avec $R^4$ étant supprimée pour $R^2$,

Z forme conjointement avec $R^3$ le reste

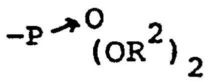

et

$R^1$ représente en outre le reste

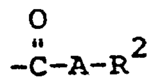

à côté de la définition mentionnée;

de formule générale III

III 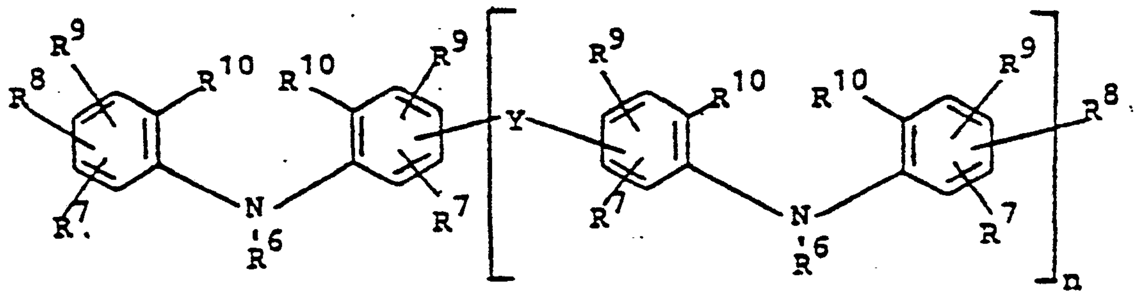

dans laquelle

$R^6$ représente H ou $R^1$,

$R^7$, $R^9$ et $R^{10}$ sont identiques ou différents et représentent H, $CH_3$ ou $C_2H_5$,

$R^8$ représente H, un reste benzyle, styryle, α-méthylstyryle, tertiobutyle, tertio-amyle, isononyle, cyclohexyle, méthylcyclohexyle,

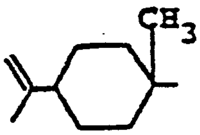, 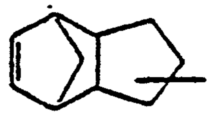, 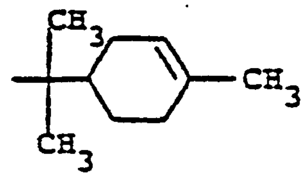, 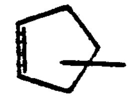

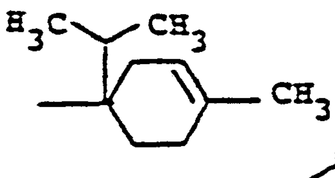, 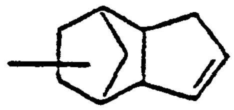, 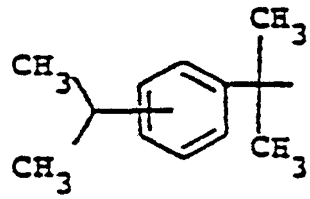,

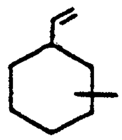, 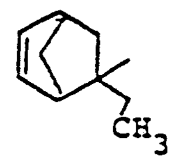

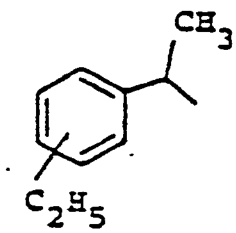, 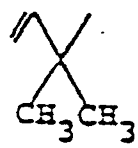, 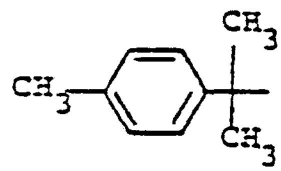,

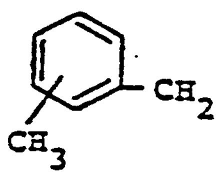 et 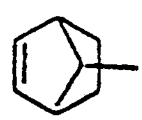

Y est un groupe de formule

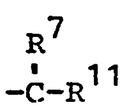 dans laquelle $R^7$ a

la définition indiquée ci-dessus et $R^{11}$ est un groupe alkyle en $C_1$ à $C_7$, cyclohexyle, cyclohexényle, aryle, en outre

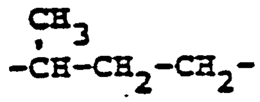, 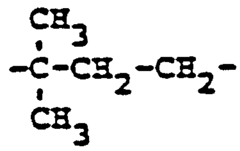, 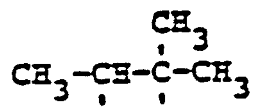,

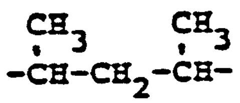, 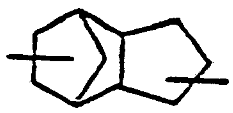, 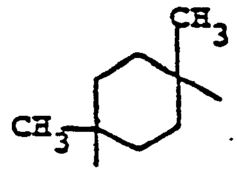, 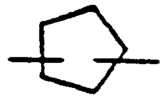,

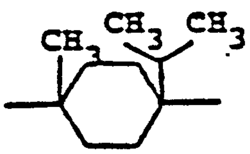, 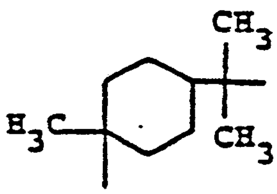, 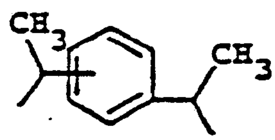, 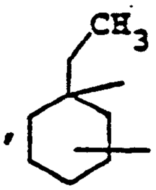,

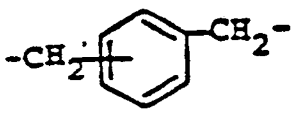, 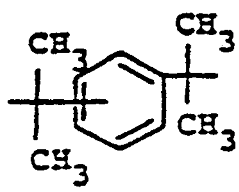, 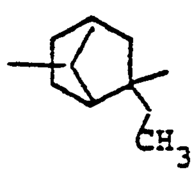

et peut aussi représenter -S-, $-CH_2-$ $-CH_2-S-CH_2-$ ou $-CH_2-O-CH_2-$ dans une proportion allant jusqu'à 60 moles %, et

n est un nombre entier de 1 à 29, mais à l'exclusion de produits correspondant à la réaction de la diphénylamine et de l'acétone ($R^6$ à $R^{10}$ = H;

Y = 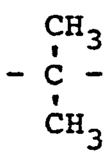 ;

de formule générale IV

IV 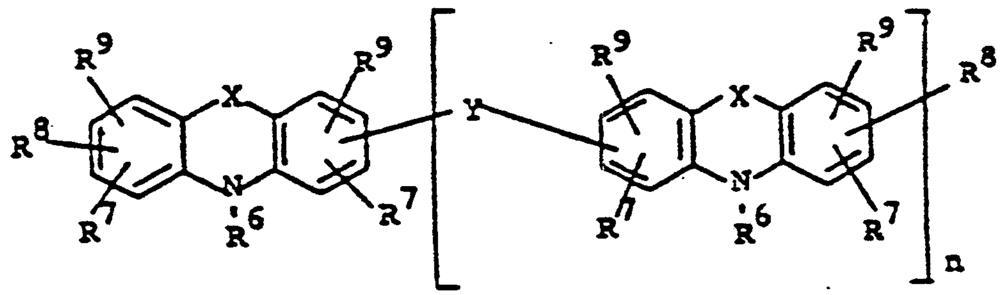

dans laquelle
X représente -S-;
et de formule générale V

V

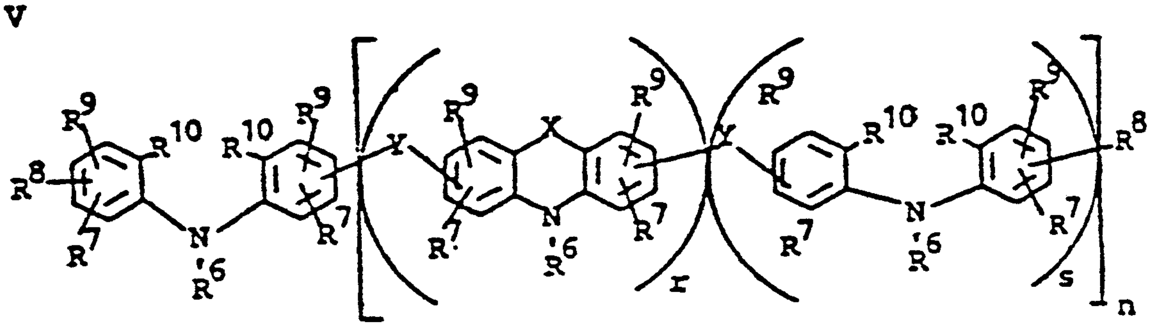

dans laquelle
r et s sont des nombres entiers de 1 à 29.
2. Composés de formule générale III

III 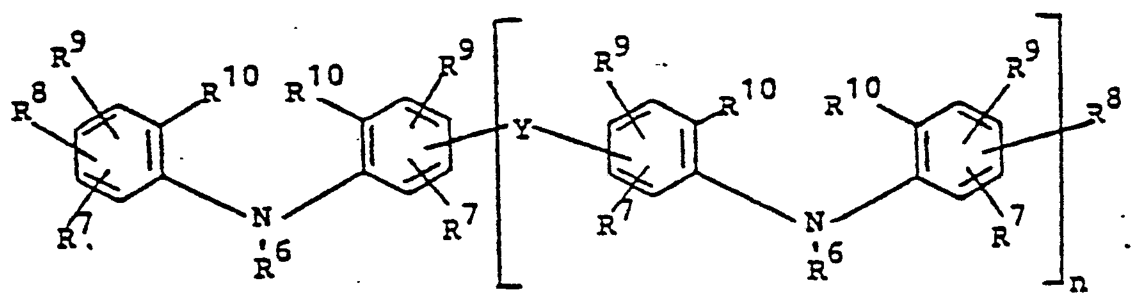

dans laquelle
$R^6$ représente H ou $R^1$,
$R^7$, $R^9$ et $R^{10}$ sont identiques ou différents et représentent H, $CH_3$ ou $C_2H_5$,
$R^8$ représente H, un reste benzyle, styryle, α-méthylstyryle, tertiobutyle, tertio-amyle, isononyle, cyclohexyle, méthylcyclohexyle,

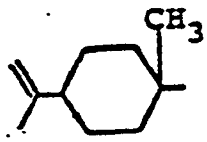 , 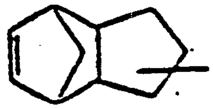 , 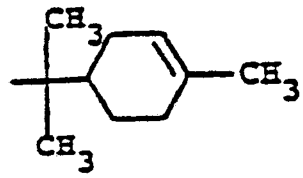 ,

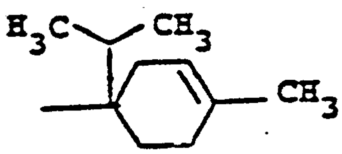 , 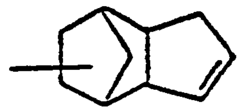 , 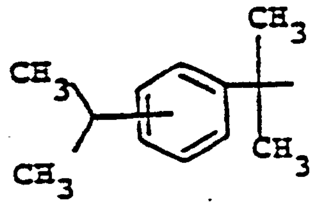 ,

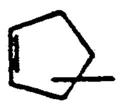 , 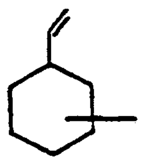 , 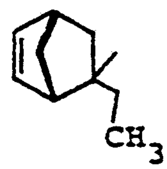

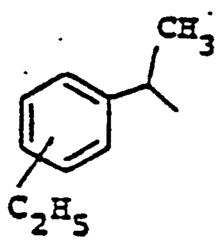 , 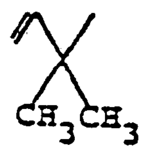 , 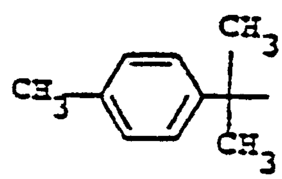 ,

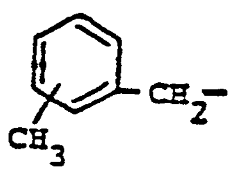 et 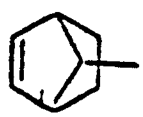

Y représente

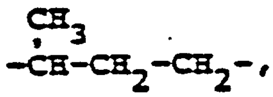, 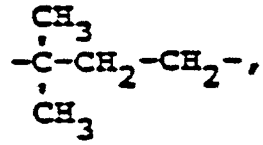, 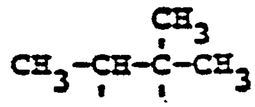,

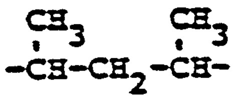, 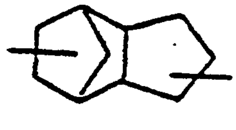, 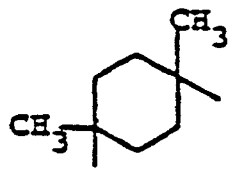, 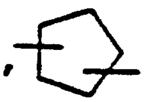,

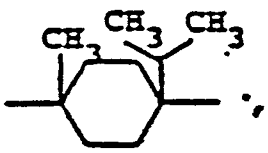, 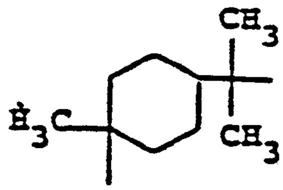, 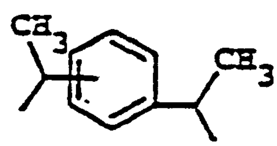, 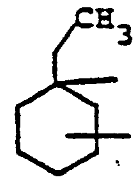,

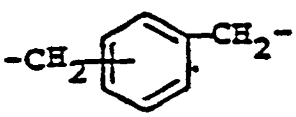, 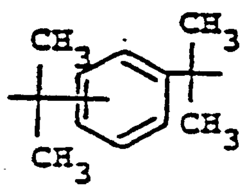, 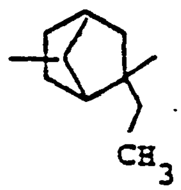

et peut aussi représenter -S-, $-CH_2-$, $-CH_2-S-CH_2-$ ou $-CH_2-O-CH_2$, dans une proportion allant jusqu'à 60 moles %, et

n est un nombre entier de 1 à 29;

mais à l'exclusion de composés dans lesquels $R^6$ à $R^{10}$ représentent H et

Y = 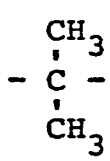 ;

de formule générale IV

IV 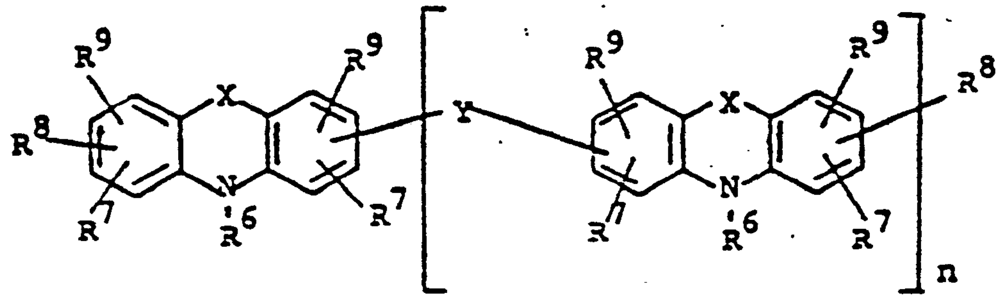

dans laquelle
Y représente

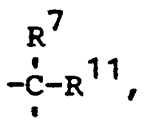

où $R^7$ a la définition donnée ci-dessus, mais représente de préférence H, et $R^{11}$ est un groupe alkyle en $C_1$-$C_7$ (de préférence en $C_1$-$C_4$), cyclohexyle, cyclohexényle et aryle, et a en outre la définition indiquée en III,
X représente - S -;
et de formule générale V

V

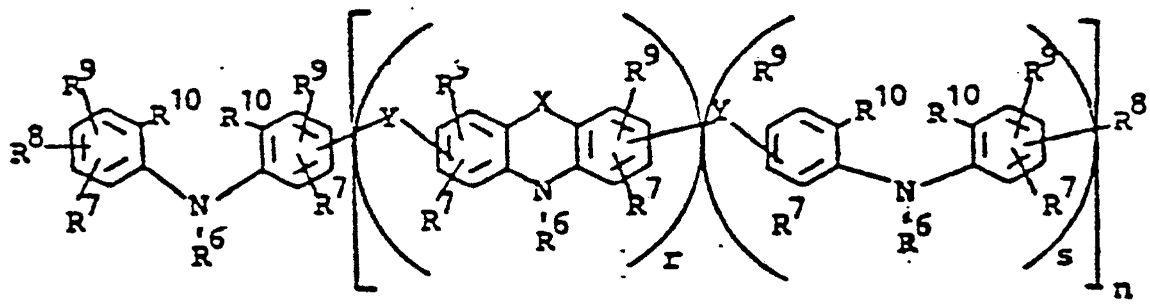

dans laquelle
r et s représentent des nombres entiers de 1 à 29.

3. Procédé de production de mélanges monomériques et/ou oligomériques des composés de formules III à V suivant la revendication 2, caractérisé en ce qu'on fait réagir des amines aromatîques de formules

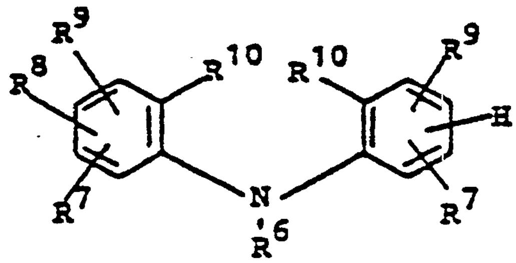

et

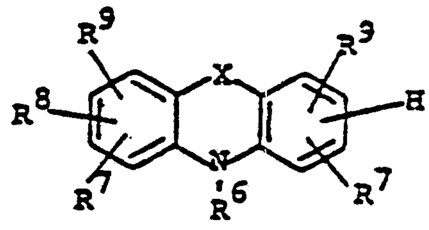

dans lesquelles $R^6$, $R^7$, $R^8$, $R^9$, $R^{10}$ et X ont la définition indiquée dans la revendication 2, avec des composés bifonctionnels de formules

Hal-Y-Hal (Hal = halogene)

HO-Y-OH

$R^7O$-Y-$OR^7$

$R^7COO$-Y-$OCOR^7$,

où le reste Y a la définition indiquée dans la revendication 2, ou avec des bisoléfines qui se forment à partir de ces composés par élimination du reste HOH, HOR, $HOCOR^7$ ou de H-Hal,

en présence d'acides forts de $pk_s$ inférieur à 2 à des températures comprises entre 50 et 300°C et les restes $R^8$ et $R^6$ (tous deux différents de H) sont introduits avant, pendant ou apres la réaction ci-dessus des amines.